(19) Europäisches Patentamt / European Patent Office / Office européen des brevets

(11) EP 3 858 632 B1

(12) EUROPÄISCHE PATENTSCHRIFT

(45) Veröffentlichungstag und Bekanntmachung des Hinweises auf die Patenterteilung:
**18.01.2023 Patentblatt 2023/03**

(21) Anmeldenummer: **21165462.9**

(22) Anmeldetag: **06.07.2017**

(51) Internationale Patentklassifikation (IPC):
***B41M 5/323*** *(2006.01)* ***B41M 5/333*** *(2006.01)*
*B41M 5/337* *(2006.01)*

(52) Gemeinsame Patentklassifikation (CPC):
**C07C 311/60; B41M 5/323; B41M 5/3333;**
B41M 5/3375

# (54) WÄRMEEMPFINDLICHES AUFZEICHNUNGSMATERIAL

HEAT-SENSITIVE RECORDING MATERIAL

MATÉRIAU D'ENREGISTREMENT THERMOSENSIBLE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **18.07.2016 DE 102016113203**

(43) Veröffentlichungstag der Anmeldung:
**04.08.2021 Patentblatt 2021/31**

(62) Dokumentnummer(n) der früheren Anmeldung(en) nach Art. 76 EPÜ:
**17737776.9 / 3 484 848**

(73) Patentinhaber: **Koehler Paper SE**
**77704 Oberkirch (DE)**

(72) Erfinder:
- **HORN, Michael**
  **77654 Offenburg (DE)**
- **ZIERINGER, Kerstin**
  **77855 Achern (DE)**

(74) Vertreter: **Held, Stephan**
**Meissner Bolte Patentanwälte**
**Rechtsanwälte Partnerschaft mbB**
**Widenmayerstraße 47**
**80538 München (DE)**

(56) Entgegenhaltungen:
**EP-A1- 0 701 905** **JP-A- H0 858 242**
**US-A1- 2004 029 056**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).



Processed by Luminess, 75001 PARIS (FR)

## Beschreibung

Die Erfindung betrifft einen Farbentwickler, ein wärmeempfindliches Aufzeichnungsmaterial, umfassend ein Trägersubstrat sowie eine mindestens einen Farbbildner und mindestens diesen Farbentwickler enthaltende wärmeempfindliche farbbildende Schicht, ein Verfahren zu dessen Herstellung sowie die Verwendung des im wärmeempfindlichen Aufzeichnungsmaterial enthaltenen phenolfreien Farbentwicklers.

Wärmeempfindliche Aufzeichnungsmaterialien für die thermodirekte Druckanwendung, die eine auf einem Trägersubstrat aufgebrachte wärmeempfindliche farbbildende Schicht (Thermoreaktionsschicht) aufweisen, sind seit langem bekannt. In der wärmeempfindlichen farbbildenden Schicht liegen üblicherweise ein Farbbildner und ein Farbentwickler vor, die unter Wärmeeinwirkung miteinander reagieren und so zu einer Farbentwicklung führen. Weit verbreitet sind preisgünstige phenolische Farbentwickler, wie beispielsweise Bisphenol A und Bisphenol S, mit welchen wärmeempfindliche Aufzeichnungsmaterialien erhalten werden können, die für zahlreiche Anwendungen ein akzeptables Leistungsprofil aufweisen. Ebenfalls bekannt sind wärmeempfindliche Aufzeichnungsmaterialien, die in der wärmeempfindlichen farbbildenden Schicht nicht-phenolische Farbentwickler enthalten. Diese wurden entwickelt, um die Beständigkeit des Schriftbildes zu verbessern, insbesondere auch dann, wenn das bedruckte wärmeempfindliche Aufzeichnungsmaterial über längere Zeit gelagert wird oder mit hydrophoben Stoffen, wie weichmacherhaltigen Materialien oder Ölen, in Kontakt kommt. Vor dem Hintergrund der öffentlichen Diskussionen über das toxische Potenzial von (bis)phenolischen Chemikalien ist das Interesse an nicht-phenolischen Farbentwicklern stark angestiegen. Hierbei war es Ziel, die Nachteile der phenolischen Farbentwickler zu vermeiden, allerdings sollten die technischen Leistungseigenschaften, die mit phenolischen Farbentwicklern erzielt werden können, zumindest beibehalten, vorzugsweise aber verbessert werden.

Der Stand der Technik zu nicht-phenolischen Farbentwicklern lässt trotz der großen chemischen Diversität dieser Stoffe gemeinsame strukturelle Merkmale erkennen.

So ist eine 1,3-disubstituierte Ureido-Struktur (Y-NH-CO-NH-Z) ein gemeinsames Merkmal zahlreicher nicht-phenolischer Farbentwickler. Durch passende Wahl der Gruppen Y und Z können die für die Eignung als Farbentwickler wesentlichen funktionellen Eigenschaften, insbesondere die Acidität und die Wasserstoffbrückendonor-Eigenschaften, moduliert werden.

Weit verbreitet sind Farbentwickler mit Sulfonyl-Harnstoff-Strukturen $-SO_2-NH-CO-NH-$, da diese relativ leicht herstellbar sind und die mit ihnen hergestellten wärmeempfindlichen Aufzeichnungsmaterialien relativ gute anwendungstechnische Eigenschaften aufweisen.

Die EP 0 526 072 A1 offenbart Farbentwickler aus der Klasse der aromatischen Sulfonyl-(thio)Harnstoffverbindungen mit der Formel

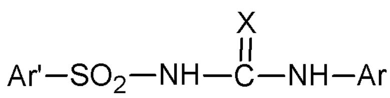

,

wobei X O oder S ist.

Mit diesen können wärmeempfindliche Aufzeichnungsmaterialien erhalten werden, die sich durch eine verbesserte Bildbeständigkeit auszeichnen. Ferner weisen die auf diesen Farbentwicklern basierenden wärmeempfindlichen Aufzeichnungsmaterialien eine brauchbare thermische Druckempfindlichkeit bei guter Oberflächenweiße auf, so dass es bei entsprechender Gestaltung der Rezeptur der wärmeempfindlichen farbbildenden Schicht vergleichsweise leicht möglich ist, hohe Druckdichten unter Verwendung von handelsüblichen Thermodruckern zu erzeugen.

Die WO 0 035 679 A1 offenbart aromatische und heteroaromatische Sulfonyl(thio)harnstoffverbindungen (X= S oder O) und/oder Sulfonyl-Guanidine (X= NH) der obigen Formel, wobei Ar durch eine zweiwertige Linkergruppe an weitere aromatische Gruppen geknüpft ist. Ein in der Praxis verbreiteter nicht-phenolischer Farbentwickler aus dieser Klasse, 4-Methyl-N-[[[3-[[(4-methylphenyl)sulfonyl] oxy]phenyl]amino]carbonyl]-benzolsulfonamid (Handelsname Pergafast 201®, BASF), zeichnet sich durch Ausgewogenheit der anwendungstechnischen Eigenschaften der mit diesem Farbentwickler hergestellten wärmeempfindlichen Aufzeichnungsmaterialien aus. Insbesondere besitzen diese eine gute dynamische Ansprechempfindlichkeit und eine hohe Beständigkeit des Ausdrucks gegenüber hydrophoben Stoffen.

Auch über eine zwei- oder mehrwertige Linkergruppe A verbunden Sulfonylharnstoffeinheiten, zum Beispiel Bis-Sulfonylharnstoffverbindungen, der Formel

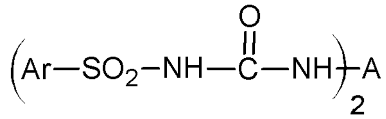

sind vielfach als Farbentwickler beschrieben worden (siehe EP 0 535 887 A1, EP 0 542 556 A1, EP 0 604 832 B1, EP 0 620 122 A1 und EP 1 044 824 A2).

In der Praxis hat sich vor allem N,N'-[Methylenbis(4,1-phenyleniminocarbonyl)]bis[4-methyl-benzolsulfonamid] (B-TUM) durchgesetzt.

Die Kombination einer Sulfonyl-Harnstoff-Struktur mit einer N-Sulfonyl-(thiol)Urethan-Gruppe der Formel

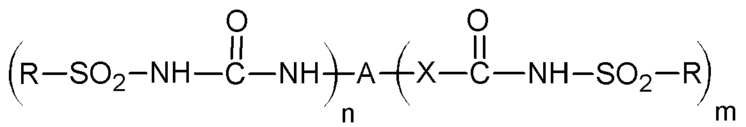

,

wobei R ein aromatischer Rest, A eine (m+n)-wertige organische Linker-Gruppe und X= O oder S ist, ist Gegenstand der JP H 0 664 335.

Für die mit diesen Farbentwickler hergestellten wärmeempfindlichen Aufzeichnungsmaterialien wird eine gesteigerte Beständigkeit des Schriftbildes gegenüber hydrophoben Agenzien beschrieben. Allerdings ist der synthetische Zugang zu diesen Farbentwicklern dann problematisch, wenn chemisch einheitliche Stoffe erwünscht sind.

Die JP H 0 958 242 kombiniert Sulfonylharnstoff-Substrukturen mit primären Sulfonamid-Gruppen, um Entwickler der Formel

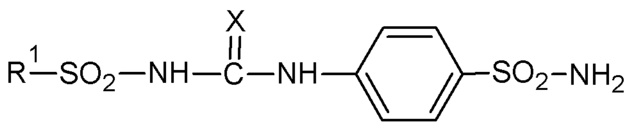

zu erhalten.

Die JP H 11-263067 offenbart Farbentwickler aus über eine aromatische Linkereinheit verbundenen (Thio)Harnstoff- und Sulfonyl-(thio)Harnstoff-Substrukturen der Formel

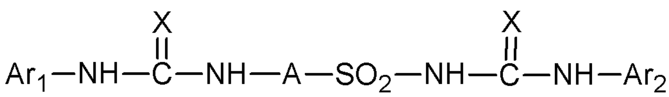

Die EP 0 701 905 A1 offenbart einen wärmeempfindlichen Farbentwickler, der eine aromatische Verbindung mit mindestens einer Gruppe der Formel -SO2NHCXNH-, wobei X ein O- oder S-Atom ist, und mindestens einer geradkettigen Alkylgruppe mit 11 oder mehr Kohlenstoffatomen umfasst und mit einem Farbstoffvorläufer in einem wärmeempfindlichen Aufzeichnungsmaterial reaktiv ist

Den auf der Sulfonylharnstoff-Chemie basierten mit nicht-phenolischen Farbentwickler erhaltenen wärmeempfindlichen Aufzeichnungsmaterialen ist gemein, dass sie in manchen anwendungsrelevanten Eigenschaften gute Leistungen zeigen, in anderen jedoch Schwächen offenbaren.

So geht häufig beispielsweise eine hohe Beständigkeit der Schrift gegenüber hydrophoben Stoffen mit einer bescheidenen Ansprechempfindlichkeit (dynamische Sensitivität) im Thermodrucker einher, welche effektiv nur durch Zuhilfenahme von großen Mengen zum Teil ganz spezieller Schmelzhilfsmittel (spezielle thermische Lösemittel, spezielle Sensibilisierungsmittel) verbessert werden kann.

Andererseits erreicht man mit bestimmten nicht-phenolischen Farbentwicklern relativ leicht hohe dynamische Sensitivitätswerte, wobei aber die Beständigkeit des Schriftbildes bescheiden ist. Durch Zuhilfenahme von Alterungsschutzmittel (Stabilisatoren) kann dieser Mangel behoben werden, allerdings auf Kosten einer komplexeren und teureren Rezeptur der Aufzeichnungsschicht.

Aufgabe der vorliegenden Erfindung ist es daher, vorstehend geschilderte Nachteile des Standes der Technik zu beheben. Insbesondere besteht die Aufgabe der vorliegenden Erfindung darin, einen Farbentwickler und ein diesen enthaltendes wärmeempfindliches Aufzeichnungsmaterial bereitzustellen, welches ein ausgewogenes anwendungstechnisches Eigenschaftsprofil hat und mindestens die Leistungen der auf bekannten nicht-phenolischen Farbentwicklern beruhenden wärmeempfindlichen Aufzeichnungsmaterialen erreicht, ohne auf spezielle Rezepturbestandteile in der wärmeempfindlichen Funktionsschicht, wie Alterungsschutzmittel, oder spezielle Schmelzhilfsmittel mit eingeschränkter Verfügbarkeit und/oder hohem Preis, angewiesen zu sein. Eine vornehmliche Aufgabe der Erfindung war es, Farbentwickler bereitzustellen, die die Ausbildung sehr stabiler Farbkomplexe ermöglichen und dadurch eine deutlich höhere Beständigkeit des Schriftbildes des wärmeempfindlichen Aufzeichnungsmaterials im Vergleich zum Stand der Technik ermöglichen.

Erfindungsgemäß wird diese Aufgabe durch den Einsatz einer Verbindung nach Anspruch 1 in einem wärmeempfindlichen Aufzeichnungsmaterial nach Anspruch 1 gelöst.

Die Verbindung nach Anspruch 1 besitzt die Formel (I),

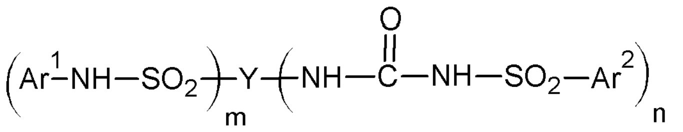

(I),

wobei m und n unabhängig voneinander ≥ 1 sind, $Ar^1$ ein unsubstituierter oder substituierter (hetero) aromatischer Rest ist, $Ar^2$ ein unsubstituierter oder substituierter Phenyl-Rest ist und Y mindestens eine (m+n)-fach substituierte Benzol- oder Naphthalin-Gruppe ist.

Vorzugsweise ist m 1 oder 2, besonders bevorzugt 1.

Vorzugsweise ist n 1 oder 2, besonders bevorzugt 1.

Vorzugsweise ist m 1 und n 1, m 1 und n 2 oder m 2 und n 2.

Vorzugsweise ist $Ar^1$ ein unsubstituierter Phenyl-, ein unsubstituierter 1-Naphtyl-, ein unsubstituierter 2-Naphtyl-, ein einfach substituierter Phenyl-, ein einfach substituierter 1-Naphtyl- oder ein einfach substituierter 2-Naphtyl-Rest. Besonders bevorzugt ist $Ar^1$ ein unsubstituierter Phenyl-Rest oder ein einfach substituierter Phenyl-Rest. Ganz besonders bevorzugt ist $Ar^1$ ein unsubstituierter Phenyl-Rest oder ein 4-Alkoxycarbonylphenyl-Rest. Ersterer ist leicht herstellbar, letzterer liefert besonders gute Ergebnisse.

Vorzugsweise ist der substituierte (hetero) aromatische Rest, insbesondere der substituierte Phenyl-Rest, mit einem $C_1$-$C_5$-Alkyl-, einem Alkenyl-, einem Alkinyl-, einem Benzyl-, einem RO-, einem Halogen-, einem ROC-, einem $RO_2C$-, einem CN-, einem $NO_2$-, einem R-$SO_2$O-, einem RO-$SO_2$-, einem R-NH- $SO_2$-, einem R-$SO_2$NH-, einem R-NH-CO-NH-, einem R-$SO_2$-NH-CO-NH-, einem R-NH-CO-NH-$SO_2$- oder einem R-CO-NH-Rest, wobei R ein $C_1$-$C_5$-Alkyl-, ein Alkenyl-, ein Alkinyl-, ein Phenyl-, ein Tolyl- oder ein Benzyl-Rest, bevorzugt ein Phenyl- oder p-Tolyl-Rest, substituiert. Bevorzugte Substituenten sind $C_1$-$C_5$-Alkyl-, RO-, Halogen-, $RO_2C$-, R-$SO_2$O-, R-NH-CO-NH- und R-$SO_2$-NH-CO-NH-Reste.

$Ar^1$ kann auch ein mehrfach substituierter (hetero) aromatischer Rest sein.

Vorzugsweise ist $Ar^2$ ein unsubstituierter Phenyl- oder ein einfach substituierter Phenyl-Rest. Der einfach substituierte Phenyl-Rest ist vorzugsweise mit einem $C_1$-$C_4$-Alkyl-Rest, besonders bevorzugt mit einem Methyl-Rest und ganz besonders bevorzugt mit einem 4-Methyl-Rest, oder mit einem Halogen-Rest substituiert.

Besonders bevorzugt ist Y eine (m+n)-fach substituierte Benzol-Gruppe. Besonders bevorzugt beträgt die Summe aus (m+n) 2, 3 oder 4, insbesondere 2, d.h. Y ist eine Phenylen-Gruppe, da derartige Verbindungen wirtschaftlich günstig herstellbar sind.

In einer bevorzugten Ausführungsform ist $Ar^1$ ein unsubstituierter Phenyl-Rest oder ein einfach substituierter Phenyl-Rest, $Ar_2$ ein unsubstituierter Phenyl-Rest oder ein einfach substituierter Phenyl-Rest und Y eine (m+n)-fach substituierte Benzol-Gruppe.

Besonders bevorzugte Verbindungen der Formel (I) sind in folgender Tabelle 1 dargestellt.

Tabelle 1: Bevorzugte Verbindungen der Formel (I) mit den angegebenen Bedeutungen für die Y-Gruppe, den $Ar^1$-Rest, den $Ar^2$-Rest, m und n (R= wie vorstehend erwähnt)

| Y | $Ar^1$ | $Ar^2$ | m | n |
|---|---|---|---|---|
| | | | | |
| Phenylen- | Phenyl- | Phenyl- | 1 | 1 |
| Phenylen- | $C_1$-$C_5$-Alkylsubstituierter Phenyl- | Phenyl- | 1 | 1 |
| Phenylen- | RO-substituierter Phenyl- | Phenyl- | 1 | 1 |
| Phenylen- | $RO_2C$-substituierter Phenyl- | Phenyl- | 1 | 1 |
| Phenylen- | Halogen substituierter Phenyl- | Phenyl- | 1 | 1 |
| Phenylen- | R-NH -CO-NH -su bstitu ierter Phenyl- | Phenyl- | 1 | 1 |

(fortgesetzt)

| Y | $Ar^1$ | $Ar^2$ | m | n |
|---|---|---|---|---|
| Phenylen- | Phenyl- | $C_1$-$C_4$-Alkylsubstituierter Phenyl- | 1 | 1 |
| Phenylen- | $C_1$-$C_5$-Alkylsubstituierter Phenyl- | $C_1$-$C_4$-Alkylsubstituierter Phenyl- | 1 | 1 |
| Phenylen- | RO-substituierter Phenyl- | $C_1$-$C_4$-Alkylsubstituierter Phenyl- | 1 | 1 |
| Phenylen- | $RO_2C$-substituierter Phenyl- | $C_1$-$C_4$-Alkylsubstituierter Phenyl- | 1 | 1 |
| Phenylen- | Halogen substituierter Phenyl- | $C_1$-$C_4$-Alkylsubstituierter Phenyl- | 1 | 1 |
| Phenylen- | R-NH -CO-NH -su bstitu ierter Phenyl- | $C_1$-$C_4$-Alkylsubstituierter Phenyl- | 1 | 1 |
| Dreifachsubstituierte Benzol- | Phenyl- | Phenyl- | 1 | 2 |
| Dreifachsubstituierte Benzol- | Phenyl- | $C_1$-$C_4$-Alkylsubstituierter Phenyl- | 1 | 2 |
| Vierfachsubstituierte Benzol- | Phenyl- | Phenyl- | 2 | 2 |
| Vierfachsubstituierte Benzol- | Phenyl- | $C_1$-$C_4$-Alkylsubstituierter Phenyl- | 2 | 2 |
| Vierfachsubstituierte Benzol- | $RO_2C$-substituierter Phenyl- | $C_1$-$C_4$-Alkylsubstituierter Phenyl- | 2 | 2 |

Die Herstellung der erfindungsgemäßen Verbindung der Formel (I) kann nach an sich bekannten Methoden erfolgen. Folgendes Reaktionsschema veranschaulicht einen möglichen Syntheseweg für die erfindungsgemäße Verbindung der Formel (I) am Beispiel der Verbindungen I-XXXII (siehe Tabelle 2).

8

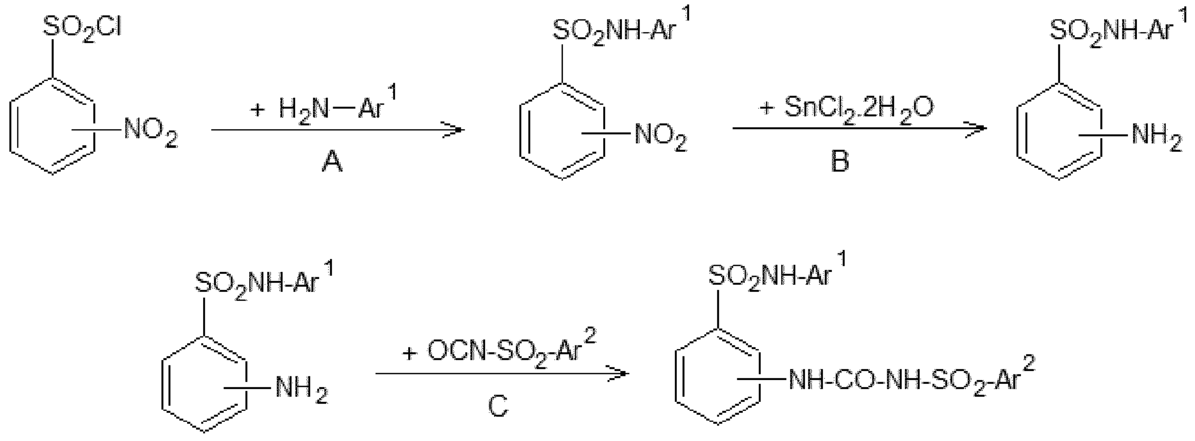


Die unter die erfindungsgemäße Verbindung der Formel (I) fallenden Verbindungen XXXIII-XXXIV (siehe Tabelle 2) können gemäß nachfolgendem Reaktionsschema ausgehend von dem entsprechenden Bis-Aminsulfonylchloriden (G. Barnikow, K.Krüger, G. Hilgetag, Z. Chem., 6,(7), 262 (1966)) in das Bis-Aminobenzolbissulfonanilid überführt und dann zum Endprodukt weiter verarbeitet werden.

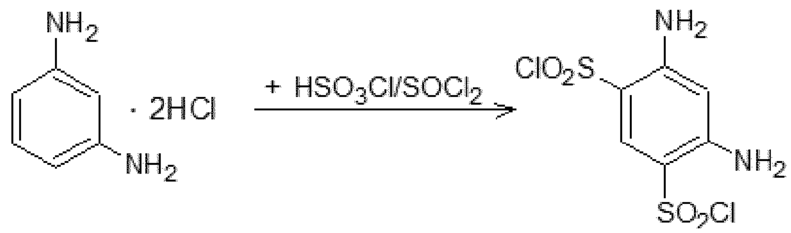


Die im Zusammenhang mit der Verbindung der Formel (I) aufgeführten bevorzugten Ausführungsformen gelten ebenfalls für das Verfahren zu dessen Herstellung.

Wie vorstehend bereits erwähnt, betrifft die vorliegende Erfindung ein wärmeempfindliches Aufzeichnungsmaterial, umfassend ein Trägersubstrat sowie eine mindestens einen Farbbildner und einen phenolfreien Farbentwickler enthaltende wärmeempfindliche farbbildende Schicht, wobei der mindestens eine phenolfreie Farbentwickler die Verbindung der vorstehend beschriebenen Formel (I) ist.

Die Verbindung der Formel (I) liegt vorzugsweise in einer Menge von etwa 3 bis etwa 35 Gew.-%, besonders bevorzugt in einer Menge von etwa 10 bis etwa 25 Gew.-%, bezogen auf den gesamten Feststoffgehalt der wärmeempfindlichen Schicht, vor.

Die Auswahl des Trägersubstrates ist nicht kritisch. Allerdings ist es bevorzugt, als Trägersubstrat Papier, synthetisches Papier und/oder eine Kunststoff-Folie einzusetzen.

Gegebenenfalls liegt zwischen dem Trägersubstrat und der wärmeempfindlichen Schicht mindestens eine weitere Zwischenschicht vor. Auch kann mindestens eine Schutzschicht und/oder mindestens eine die Bedruckbarkeit oder die Barriereigenschaften begünstigende Schicht im erfindungsgemäßen wärmeempfindlichen Aufzeichnungsmaterial vorliegen, wobei diese Schichten auf der Vorder- oder Rückseite des Substrats aufgebracht werden können.

Hinsichtlich der Wahl des Farbbildners unterliegt die vorliegende Erfindung ebenfalls keinen wesentlichen Einschränkungen. Bevorzugt ist der Farbbildner jedoch ein Farbstoff vom Triphenylmethan-Typ, vom Fluoran-Typ, vom Azaphthalid-Typ und/oder vom Fluoren-Typ. Ein ganz besonders bevorzugter Farbbildner ist ein Farbstoff vom Fluoran-Typ, da er dank der Verfügbarkeit und der ausgewogenen anwendungsbezogenen Eigenschaften die Bereitstellung eines Aufzeichnungsmaterials mit einem attraktiven Preis-Leistungsverhältnis ermöglicht.

Besonders bevorzugte Farbstoffe vom Fluoran-Typ sind:

3-Diethylamino-6-methyl-7-anilinofluoran,
3-(N-ethyl-N-p-toludinamino)-6-methyl-7-anilinofluoran,
3-(N-ethyl-N-isoamylamino)-6-methyl-7-anilinofluoran,
3-Diethylamino-6-methyl-7-(o,p-dimethylanilino)fluoran,
3-Pyrrolidino-6-methyl-7-anilinofluoran,
3-(Cyclohexyl-N-methylamino)-6-methyl-7-anilinofluoran,
3-Diethylamin-7-(m-trifluoromethylanilino)fluoran,
3-N-n-Dibutylamin-6-methyl-7-anilinofluoran,
3-Diethylamino-6-methyl-7-(m-methylanilino)fluoran,
3-N-n-dibutylamin-7-(o-chloroanilino) fluoran,
3-(N-Ethyl-N-tetrahydrofurfurylamin)-6-methyl-7-anilino-fluoran,
3-(N-methyl-N-propylamin)-6-methyl-7-anilinofluoran,
3-(N-ethyl-N-ethoxypropylamin)-6-methyl-7-anilinofluoran,
3-(N-ethyl-N-isobutylamin)-6-methyl-7-anilinofluoran und/oder
3-Dipentylamin-6-methyl-7-anilinfluoran.

Die Farbbildner können als Einzelstoffe, wie auch als beliebige Gemische von zwei oder mehreren Farbbildnern zur Anwendung kommen, vorausgesetzt die wünschenswerten anwendungstechnischen Eigenschaften der Aufzeichnungsmaterialien leiden darunter nicht.

Der Farbbildner liegt vorzugsweise in einer Menge von etwa 5 bis etwa 30 Gew.%, besonders bevorzugt in einer Menge von etwa 8 bis etwa 20 Gew.%, bezogen auf den gesamten Feststoffgehalt der wärmeempfindlichen Schicht, vor.

Zur Steuerung spezieller anwendungstechnischer Eigenschaften kann es vorteilhaft sein, wenn mindestens zwei unter die Formel (I) fallende Verbindungen als Farbentwickler in der wärmeempfindlichen Schicht vorliegen. Desgleichen können ein oder mehrere weitere (bis)phenolische oder nicht-phenolische Farbentwickler zusätzlich zu Verbindungen der Formel (I) in der wärmeempfindlichen farbbildenden Schicht vorliegen.

Neben dem mindestens einem Farbbildner und dem mindestens einem Farbentwickler können in der wärmeempfindlichen farbbildenden Schicht ein oder mehrere Sensibilisierungsmittel (auch thermische Lösungsmittel) genannt, vorliegen, was den Vorteil hat, dass die Steuerung der thermischen Druckempfindlichkeit leichter zu realisieren ist.

Generell kommen als Sensibilisierungsmittel vorteilhafterweise kristalline Stoffe in Betracht, deren Schmelzpunkt zwischen etwa 90 und etwa 150°C liegt und die im geschmolzenen Zustand die farbbildenden Komponenten (Farbbildner und Farbentwickler) lösen, ohne die Bildung des Farbkomplexes zu stören.

Vorzugsweise ist das Sensibilisierungsmittel ein Fettsäureamid, wie Stearamid, Beheneamid oder Palmitamid, ein Ethylen-bis-Fettsäureamid, wie N,N'-Ethylen-bis-stearinsäureamid oder N,N'-Ethylen-bis-ölsäureamid, ein Fettsäurealkanolamid , wie N-(Hydroxymethyl)stearamid, N-Hydroxymethylpalmitamid oder Hydroxyethylstearamid, ein Wachs, wie Polyethylenwachs oder Montanwachs, ein Carbonsäureester, wie Dimethylterephthalat, Dibenzylterephthalat, Benzyl-p-benzyloxybenzoat, Di-(p-methylbenzyl)oxalat, Di-(p-chlorbenzyl)oxalat oder Di-(p-benzyl)oxalat, ein aromatischer Ether, wie 1,2-Diphenoxy-ethan, 1,2-Di-(3-methylphenoxy)ethan, 2-Benzyloxynaphthalin oder 1,4-Diethoxynaphthalin, ein aromatisches Sulfon, wie Diphenylsulfon, und/oder ein aromatisches Sulfonamid, wie Benzolsulfonanilid oder N-Benzyl-p-toluolsulfonamid.

Das Sensibilisierungsmittel liegt vorzugsweise in einer Menge von etwa 10 bis etwa 40 Gew.-%, besonders bevorzugt in einer Menge von etwa 15 bis etwa 25 Gew.%, bezogen auf den gesamten Feststoffgehalt der wärmeempfindlichen Schicht, vor.

In einer weiteren bevorzugten Ausführungsform liegt neben dem Farbbildner, dem phenolfreien Farbentwickler und dem Sensibilisierungsmittel optional mindestens ein Stabilisator (Alterungsschutzmittel) in der wärmeempfindlichen, farbbildenden Schicht vor.

Bei dem Stabilisator handelt es sich vorzugsweise um sterisch gehinderte Phenole, besonders bevorzugt um 1,1,3-Tris-(2-methyl-4-hydroxy-5-cyclohexyl-phenyl)-butan, 1,1,3-Tris-(2-methyl-4-hydroxy-5-tert-butylphenyl)-butan, 1,1-Bis-(2-methyl-4-hydroxy-5-tert-butyl-phenyl)-butan.

Auch Harnstoff-Urethan-Verbindungen der allgemeinen Formel (II), Handelsprodukt UU (Urea-Urethane), oder vom 4,4'-Dihydroxydiphenylsulfon abgeleitete Ether, wie 4-Benzyloxy-4'-(2-methylglycidyloxy)-diphenylsulfon (Handelsname NTZ-95®, Nippon Soda Co. Ltd.), oder oligomere Ether der allgemeinen Formel (III) (Handelsname D90®, Nippon Soda Co. Ltd.) sind als Stabilisatoren im erfindungsgemäßen Aufzeichnungsmaterial einsetzbar.

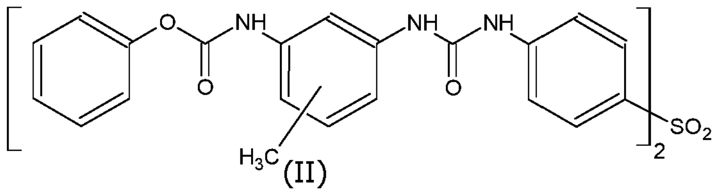

(II)

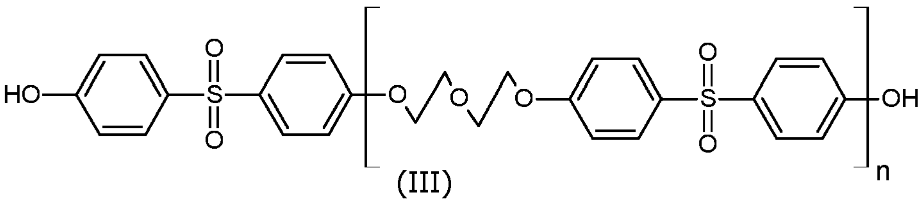

(III)

Besonders bevorzugt sind die Harnstoff-Urethan-Verbindungen der allgemeinen Formel (II).

Der Stabilisator liegt vorzugsweise in einer Menge von 0,2 bis 0,5 Gew.-Teilen, bezogen auf den mindestens einen phenolfreien Farbentwickler der Verbindung der Formel (I), vor.

In einer weiteren bevorzugten Ausführungsform liegt in der wärmeempfindlichen farbbildenden Schicht mindestens ein Bindemittel vor. Bei diesem handelt es sich vorzugsweise um wasserlösliche Stärken, Stärkederivate, stärkebasierte Biolatices vom EcoSphere® -Typ, Methylcellulose, Hydroxyethylcellulose, Carboxymethylcellulosen, partiell- oder vollständig verseifte Polyvinylalkohole, chemisch modifizierte Polyvinylalkohole oder Styrolmaleinsäureanhydrid-Copolymere, Styrolbutadien-Copolymere, Acrylamid-(Meth)-acrylat-Copolymere, Acrylamid-Acrylat-Methacrylat-Terpolymere, Polyacrylate, Poly(meth)-acrylsäureester, Acrylat-Butadien-Copolymere, Polyvinylacetate und/oder Acrylnitril-Butadien-Copolymere.

In einer weiteren bevorzugten Ausführungsform liegt mindestens ein Trennmittel (Antihaftmittel) oder Gleitmittel in der wärmeempfindlichen farbbildenden Schicht vor. Bei diesen Mitteln handelt es sich vorzugsweise um Fettsäure-Metallsalze, wie z.B. Zinkstearat oder Calciumstearat, oder auch Behenatsalze, synthetische Wachse, z.B. in Form von Fettsäureamiden, wie z.B. Stearinsäureamid und Behensäureamid, Fettsäurealkanolamide, wie z.B. Stearinsäure-methylolamid, Paraffinwachse verschiedener Schmelzpunkte, Esterwachse unterschiedlicher Molekulargewichte, Ethylenwachse, Propylenwachse unterschiedlicher Härten und/oder natürliche Wachse, wie z.B. Carnaubawachs oder Montanwachs.

Das Trennmittel liegt vorzugsweise in einer Menge von etwa 1 bis etwa 10 Gew.-%, besonders bevorzugt in einer Menge von etwa 3 bis etwa 6 Gew.-%, bezogen auf den gesamten Feststoffgehalt der wärmeempfindlichen Schicht, vor.

In einer weiteren bevorzugten Ausführungsform enthält die wärmeempfindliche farbbildende Schicht Pigmente. Der Einsatz dieser hat unter anderem den Vorteil, dass diese auf ihrer Oberfläche die im thermischen Druckprozess entstehende Chemikalien-Schmelze fixieren können. Auch kann über Pigmente die Oberflächenweisse und Opazität der wärmeempfindlichen farbbildenden Schicht und deren Bedruckbarkeit mit konventionellen Druckfarben gesteuert werden. Schließlich besitzen Pigmente eine "Extenderfunktion", beispielsweise für die relativ teuren farbgebenden Funktionschemikalien.

Besonders geeignete Pigmente sind anorganische Pigmente, sowohl synthetischer als auch natürlicher Herkunft, vorzugsweise Clays, gefällte oder natürliche Calciumcarbonate, Aluminiumoxide, Aluminiumhydroxide, Kieselsäuren, gefällte und pyrogene Kieselsäuren (z. B. nanoskalige Qualitäten, wie Aerodisp®-Typen), Diathomeenerden, Magnesiumcarbonate, Talk, aber auch organische Pigmente, wie Hohlpigmente mit einer Styrol/Acrylat-Copolymer-Wand oder Harnstoff/Formaldehyd-Kondensationspolymere. Diese können alleine oder in beliebigen Mischungen verwendet werden.

Die Pigmente liegen vorzugsweise in einer Menge von etwa 20 bis etwa 50 Gew.-%, besonders bevorzugt in einer Menge von etwa 30 bis etwa 40 Gew.-%, bezogen auf den gesamten Feststoffgehalt der wärmeempfindlichen Schicht, vor.

Zum Steuern der Oberflächenweisse des erfindungsgemäßen wärmeempfindlichen Aufzeichnungsmaterials können optische Aufheller in die wärmeempfindliche farbbildende Schicht eingebaut werden. Bei diesen handelt es sich vorzugsweise um Stilbene.

Um bestimmte streichtechnische Eigenschaften zu verbessern, ist es im Einzelfall bevorzugt zu den zwingenden Bestandteilen des erfindungsgemäßen wärmeempfindlichen Aufzeichnungsmaterials weitere Bestandteile, insbesondere Rheologie-Hilfsmittel, wie zum Beispiel Verdicker und/oder Tenside, hinzuzufügen.

Wie vorstehend erwähnt, kann die wärmeempfindliche farbbildende Schicht daher vorzugsweise übliche Additive, wie beispielsweise Sensibilisierungsmittel, Stabilisatoren, Bindemittel, Trennmittel, Pigmente und/oder Aufheller, enthalten.

Das Flächenauftragsgewicht der (trocknen) wärmeempfindlichen Schicht beträgt vorzugsweise etwa 1 bis etwa 10 $g/m^2$, bevorzugt etwa 3 bis etwa 6 $g/m^2$.

In einer besonders bevorzugten Ausführungsform handelt es sich bei dem wärmeempfindlichen Aufzeichnungsmaterial um ein solches nach Anspruch 1, wobei als Farbbildner ein Farbstoff vom Fluoran-Typ eingesetzt wird und zusätzlich ein Sensibilisierungsmittel, ausgewählt aus der Gruppe bestehend aus Fettsäureamiden, aromatischen Sulfonen und/oder aromatischen Ethern, vorliegt. In dieser bevorzugten Ausführungsform ist es auch vorteilhaft, dass etwa 1,5 bis etwa 4 Gew.-Teile des phenolfreien Farbentwicklers nach Formel (I), bezogen auf den Farbbildner, vorliegen.

Die im Zusammenhang mit der Verbindung der Formel (I) aufgeführten bevorzugten Ausführungsformen gelten ebenfalls für das erfindungsgemäße wärmeempfindliche Aufzeichnungsmaterial.

Das erfindungsgemäße wärmeempfindliche Aufzeichnungsmaterial lässt sich mit bekannten Herstellungsverfahren gewinnen.

Es ist jedoch bevorzugt, das erfindungsgemäße Aufzeichnungsmaterial mit einem Verfahren zu gewinnen, bei dem auf ein Trägersubstrat eine die Ausgangsmaterialien der wärmeempfindlichen farbbildenden Schicht enthaltende wässrige Suspension aufgetragen und getrocknet wird, wobei die wässrige Auftragssuspension einen Feststoffgehalt von etwa 20 bis etwa 75 Gew.-% , bevorzugt von etwa 30 bis etwa 50 Gew.%, aufweist, und mit dem Curtain-Coating-Beschichtungsverfahren bei einer Betriebsgeschwindigkeit der Streichanlage von mindestens etwa 400 m/min aufgetragen und getrocknet wird.

Dieses Verfahren ist insbesondere unter wirtschaftlichen Gesichtspunkten vorteilhaft.

Wird der Wert des Feststoffgehaltes von etwa 20 Gew.-% unterschritten, dann verschlechtert sich die Wirtschaftlichkeit, da eine große Menge von Wasser aus dem Strich durch schonende Trocknung in kurzer Zeit entfernt werden muss, was sich nachteilig auf die Streichgeschwindigkeit auswirkt. Wird auf der anderen Seite der Wert von 75 Gew.-% überschritten, dann führt dies lediglich zu einem erhöhten technischen Aufwand, um die Stabilität des Streichfarben-Vorhangs während des Beschichtungsprozesses zu gewährleisten.

Wie vorstehend erwähnt, ist es von Vorteil das erfindungsgemäße wärmeempfindliche Aufzeichnungsmaterial mittels eines Verfahrens herzustellen, bei dem die wässrige Auftragsuspension mit dem Curtain-Coating-Beschichtungsverfahren bei einer Betriebsgeschwindigkeit der Streichanlage von mindestens etwa 400 m/min aufgetragen wird. Das sogenannte Curtain-Coating-Verfahren ist dem Fachmann bekannt und zeichnet sich durch folgende Kriterien aus:
Beim Curtain-Coating-Beschichtungsverfahren (Vorhangbeschichtungsverfahren) wird ein frei fallender Vorhang einer Beschichtungsdispersion gebildet. Durch freien Fall wird die in Form eines dünnen Filmes (Vorhangs) vorliegende

Beschichtungsdispersion auf ein Substrat "gegossen", um die Beschichtungsdispersion auf das Substrat aufzubringen. Die DE 10196052 T1 offenbart den Einsatz des Curtain-Coating-Beschichtungsverfahrens bei der Herstellung von Informationsaufzeichnungsmaterialien u.a. auch von wärmeempfindlichen Aufzeichnungsmaterialien, wobei mehrschichtige Aufzeichnungsschichten durch Aufbringen des aus mehrere Beschichtungsdispersionsfilmen bestehenden Vorhangs auf Substrate erfolgen (Gesch. max. 200 m/min).

Die Einstellung der Betriebsgeschwindigkeit der Streichanlage auf mindestens etwa 400 m/min hat sowohl betriebswirtschaftliche als auch technische Vorteile. Besonders bevorzugt beträgt die Betriebsgeschwindigkeit mindestens etwa 750 m/min, ganz besonders bevorzugt mindestens etwa 1000 m/min und ganz besonders bevorzugt mindestens etwa 1500 m/min. Es war insbesondere überraschend, dass selbst bei letztgenannter Geschwindigkeit das erhaltene wärmeempfindliche Aufzeichnungsmaterial in keiner Weise beeinträchtigt ist und dass die Betriebsdurchführung selbst bei dieser hohen Geschwindigkeit optimal abläuft.

In einer bevorzugten Ausführungsform des erfindungsgemäßen Verfahrens weist die wässrige entlüftete Auftragssuspension eine Viskosität von etwa 150 bis etwa 800 mPas (Brookfield, 100 U/min, 20 °C) auf. Wird der Wert von etwa 150 mPas unterschritten bzw. der Wert von etwa 800 mPas überschritten, dann führt dies zu einer mangelhaften Lauffähigkeit der Streichmasse am Streichaggregat. Besonders bevorzugt beträgt die Viskosität der wässrigen entlüfteten Auftragssuspension etwa 200 bis etwa 500 mPas.

In einer bevorzugten Ausführungsform kann zur Optimierung des Verfahrens die Oberflächenspannung der wässrigen Auftragssuspension auf etwa 25 bis etwa 60 mN/m, bevorzugt auf etwa 35 bis etwa 50 mN/m (gemessen entsprechend der statischen Ringmethode nach Du Noüy, DIN 53914), eingestellt werden.

Die Ausbildung der wärmeempfindlichen farbbildenden Schicht kann on-line oder in einem separaten Streichvorgang off-line erfolgen. Dies gilt auch für eventuell nachfolgend aufgetragene Schichten oder Zwischenschichten.

Es ist vorteilhaft, wenn die getrocknete wärmeempfindliche farbbildende Schicht einer Glätt-Maßnahme unterzogen wird. Bevorzugt erfolgt die Glättung der Oberfläche des Aufzeichnungsmaterials mit einem Schuhglättwerk entsprechen DE 102004029261 B4. Hierbei ist es vorteilhaft, die Bekk-Glätte, gemessen nach ISO 5627, auf etwa 100 bis etwa 1000 sec, vorzugsweise auf etwa 250 bis etwa 600 sec, einzustellen.

Die Oberflächenrauigkeit (PPS) nach ISO 8791-4 liegt im Bereich von etwa 0,50 bis etwa 2,50 $\mu$m, vorzugsweise zwischen 1,00 -2,00 $\mu$m.

Die im Zusammenhang mit der Verbindung der Formel (I) aufgeführten bevorzugten Ausführungsformen gelten ebenfalls für das erfindungsgemäße Verfahren zur Herstellung des erfindungsgemäßen wärmeempfindlichen Aufzeichnungsmaterials.

Die vorliegende Erfindung betrifft auch ein wärmeempfindliches Aufzeichnungsmaterial, welches mit dem voranstehend geschilderten Verfahren erhältlich ist.

Das vorstehend geschilderte Verfahren ist unter wirtschaftlichen Gesichtspunkten vorteilhaft und erlaubt eine hohe Verfahrensführung der Streichanlage sogar bei einer Geschwindigkeit von mehr als 1500 m/min, ohne dass es zu Beeinträchtigungen des Verfahrenserzeugnisses, das heißt des erfindungsgemäßen wärmeempfindlichen Aufzeichnungsmaterials kommt. Die Verfahrensführung kann on-line und off-line erfolgen, was eine wünschenswerte Flexibilität zur Folge hat.

Das erfindungsgemäße wärmeempfindliche Aufzeichnungsmaterial ist phenolfrei, und für POS (point-of-sale) und/oder Etiketten-Anwendungen gut geeignet. Es eignet sich auch zur Herstellung von Parkscheinen, Fahrkarten, Eintrittskarten, Lotto- und Wettscheinen, etc., welche in Thermodirektverfahren bedruckt werden können und eine hohe Beständigkeit der darauf aufgezeichneten Bilder unter längerfristiger Lagerung, selbst unter verschärften Klimabedingungen hinsichtlich Temperatur und Umgebungsfeuchte, und des in-Kontakt-bringens des Schriftbildes mit hydrophoben Stoffen, wie Weichmachern, fettigen oder öligen Stoffen, etc., gewährleistet.

Überraschenderweise hat es sich gezeigt, dass es möglich ist mit dem Farbentwickler der Formel (I) wärmeempfindliche Aufzeichnungsmaterialien zu erhaltenen, welche sich durch eine herausragende Beständigkeit des Schriftbildes gegenüber hydrophoben Agenzien auszeichnen. Auch werden gute dynamische Druckempfindlichkeiten erreicht.

Ohne daran gebunden zu sein, wird vermutet, dass die Stabilität der mit der Verbindung bzw. dem Farbentwickler der Formel (I) erhaltene Farbkomplex dadurch erzielt wird, dass die Sulfonamid-Einheit $-SO_2-NH-Ar^1$ zusätzliche Wasserstoffbrückendonor-Kapazitäten für die Stabilisierung des Farbkomplexes bereitstellen, wobei der Möglichkeit der Modulierung die Acidität dieser Struktureinheit durch adäquat substituierte Reste $Ar^1$, eine besondere Bedeutung zukommt.

Die Erfindung wird nachfolgend anhand von nicht beschränkten Beispielen im Detail erläutert.

Beispiele:

Herstellung der erfindungsgemäßen Verbindungen der Formel (I).

Die Verbindungen I-XXXII (Tabelle 2) wurden wie folgt hergestellt:

Stufe A - Herstellung der Nitroarylsulfonanilide

Zu einem Gemisch aus 55 mmol aromatischem Amin und 50 mmol Pyridin wird unter Rühren portionsweise 50 mmol des Nitroarylsulfonylchlorids zugefügt. Das Gemisch wird kurzzeitig (5-10 min) auf 95-100°C erwärmt, abgekühlt und mit 100-150 ml Salzsäure (2mol/l) verrieben. Nach Abnutschen wird mit Waser neutral gewaschen und getrocknet. Die Nitroarylsulfonanilide wurden ohne vorherige Aufreinigung in der Stufe B verwendet.

Stufe B - Reduktion der Nitro-Gruppe zum primären Amin

40 mmol des Produktes aus Stufe A werden in 140 ml Ethylacetat gelöst, unter Rühren mit 140 mmol $SnCl_2.2H_2O$ versetzt und am Rückfluß erhitzt. Der Reaktionsverauf wird über DC verfolgt (Laufmittel Cyclohexan/Ethylacetat 1:1). Nach Beenden der Reaktion (ca. 2 - 3 h) wird mit 140 ml Ethylacetat verdünnt, mit einer 10 % wässrigen $K_2CO_3$-Lösung versetzt und 30 min bei Raumtemperatur gerührt. Die Sn-Verbindungen werden abfiltriert und im Filtrat die wässrige von der organischen Phase getrennt. Letztere wird 2 mal mit je 100 ml einer gesättigter Natriumchorid-Lösung ausgeschüttelt und anschließend über $MgSO_4$ getrocknet. Nach Abrotieren des Lösungsmittels bleibt das Aminoarylsulfanilid als Feststoff zurück. Die Aufreinigung erfolgt durch Umkristallisieren aus wässrigem Methanol.

Stufe C - Herstellung der Sulfonylharnstoff-Verbindungen

15 mmol des Produktes aus Stufe B werden in Dichlormethan (60-100 ml) gelöst und bei Raumtemperatur mit 15 mmol Sulfonylisocyanat portionsweise unter Rühren versetzt. Die Reaktion wird mittels DC verfolgt (Laufmittel n-Hexan/CHCl3/$CH_3OH$ 4:2:1). Nach Beenden der Reaktion wird das ausgefallene Produkt abfiltriert, mit Dichlormethan gewaschen und bei < 70°C getrocknet.

Die Verbindungen XXXIII - XXXIV (Tabelle 2) wurden, ausgehend aus dem entsprechenden Bis-amin sulfonylchloriden (Schema 2, G. Barnikow, K.Krüger, G. Hilgetag, Z. Chem., 6,(7), 262 (1966)), nach der allgemeinen Vorschrift von Stufe A in die Bis-Aminobenzolbissulfonanilide überführt und diese entsprechend der Vorschrift von Stufe C in die Endprodukte.

Die Herstellung der Ausgangsverbindungen ist bekannt bzw. sind diese kommerziell erhältlich.

Tabelle 2

| | Y | $Ar^1$ | $Ar^2$ | m | n |
|---|---|---|---|---|---|
| I | 1,2-Phenylen | $C_6H_5$ | $C_6H_5$ | 1 | 1 |
| II | 1,2-Phenylen | $2-CH_3-C_6H_4$ | $C_6H_5$ | 1 | 1 |
| III | 1,2-Phenylen | $4-CH_3-C_6H_4$ | $C_6H_5$ | 1 | 1 |
| IV | 1,2-Phenylen | $4-OCH_3-C_6H_4$ | $C_6H_5$ | 1 | 1 |
| v | 1,2-Phenylen | $2-(CO_2C_2H_5)-C_6H_4$ | $C_6H_5$ | 1 | 1 |
| VI | 1,2-Phenylen | $4-(CO_2CH_3)-C_6H_4$ | $C_6H_5$ | 1 | 1 |
| VII | 1,2-Phenylen | $3-Cl-C_6H_4$ | $C_6H_5$ | 1 | 1 |
| VIII | 1,2-Phenylen | $2-(NH-CO-NH-C_6H_5)-C_6H_4$ | $C_6H_5$ | 1 | 1 |
| IX | 1,2-Phenylen | $C_6H_5$ | $4-CH_3-C_6H_4$ | 1 | 1 |
| X | 1,2-Phenylen | $2-CH_3-C_6H_4$ | $4-CH_3-C_6H_4$ | 1 | 1 |
| XI | 1,2-Phenylen | $4-CH_3-C_6H_4$ | $4-CH_3-C_6H_4$ | 1 | 1 |
| XII | 1,2-Phenylen | $3-CH_3-C_6H_4$ | $4-CH_3-C_6H_4$ | 1 | 1 |
| XIII | 1,2-Phenylen | $4-OCH_3-C_6H_4$ | $4-CH_3-C_6H_4$ | 1 | 1 |
| XIV | 1,2-Phenylen | $2-(CO_2C_2H_5)- C_6H_4$ | $4-CH_3-C_6H_4$ | 1 | 1 |
| XV | 1,2-Phenylen | $4-(CO_2CH_3)-C_6H_4$ | $4-CH_3-C_6H_4$ | 1 | 1 |
| XVI | 1,2-Phenylen | $2-Cl-C_6H_4$ | $4-CH_3-C_6H_4$ | 1 | 1 |
| XVII | 1,2-Phenylen | $3-Cl-C_6H_4$ | $4-CH_3-C_6H_4$ | 1 | 1 |
| XVIII | 1,2-Phenylen | $4-Cl-C_6H_4$ | $4-CH_3-C_6H_4$ | 1 | 1 |

(fortgesetzt)

| | Y | $Ar^1$ | $Ar^2$ | m | n |
|---|---|---|---|---|---|
| XIX | 1,2-Phenylen | $4\text{-}OC_2H_5\text{-}C_6H_4$ | $4\text{-}CH_3\text{-}C_6H_4$ | 1 | 1 |
| XX | 1,2-Phenylen | $2\text{-}OCH_3\text{-}C_6H_4$ | $4\text{-}CH_3\text{-}C_6H_4$ | 1 | 1 |
| XXI | 1,2-Phenylen | $3\text{-}(O\text{-}SO_2\text{-}C_6H_4\text{-}4\text{-}CH_3)\text{-}C_6H_4$ | $4\text{-}CH_3\text{-}C_6H_4$ | 1 | 1 |
| XXII | 1,2-Phenylen | $2\text{-}(NH\text{-}CO\text{-}NH\text{-}C_6H_5)\text{-}C_6H_4$ | $4\text{-}CH_3\text{-}C_6H_4$ | 1 | 1 |
| XXIII | 1,2-Phenylen | $4\text{-}(CO_2C_2H_5)\text{-}C_6H_4$ | $4\text{-}CH_3\text{-}C_6H_4$ | 1 | 1 |
| XXIV | 1,3-Phenylen | $C_6H_5$ | $4\text{-}CH_3\text{-}C_6H_4$ | 1 | 1 |
| XXV | 1,3-Phenylen | $2\text{-}(NH\text{-}CO\text{-}NH\text{-}C_6H_5)\text{-}C_6H_4$ | $4\text{-}CH_3\text{-}C_6H_4$ | 1 | 1 |
| XXVI | 1,4-Phenylen | $C_6H_5$ | $4\text{-}CH_3\text{-}C_6H_4$ | 1 | 1 |
| XXVII | 1,4-Phenylen | $3\text{-}CH_3\text{-}C_6H_4$ | $4\text{-}CH_3\text{-}C_6H_4$ | 1 | 1 |
| XXVIII | 1,4-Phenylen | $4\text{-}(CO_2CH_3)\text{-}C_6H_4$ | $4\text{-}CH_3\text{-}C_6H_4$ | 1 | 1 |
| XXIX | 1,4-Phenylen | $4\text{-}Cl\text{-}C_6H_4$ | $4\text{-}CH_3\text{-}C_6H_4$ | 1 | 1 |
| XXX | 1,4-Phenylen | $2\text{-}(NH\text{-}CO\text{-}NH\text{-}C_6H_5)\text{-}C_6H_4$ | $4\text{-}CH_3\text{-}C_6H_4$ | 1 | 1 |
| XXXI | 1,4-Phenylen | $4\text{-}(CO_2C_2H_5)\text{-}C_6H_4$ | $4\text{-}CH_3\text{-}C_6H_4$ | 1 | 1 |
| XXXII | Benzol-1,2,4-triyl | $C_6H_5$ | $4\text{-}CH_3\text{-}C_6H_4$ | 1 | 2 |
| XXXIII | Benzol-1,3,4,6-tetryl | $C_6H_5$ | $C_6H_5$ | 2 | 2 |
| XXXIV | Benzol-1,3,4,6-tetryl | $C_6H_5$ | $4\text{-}CH_3\text{-}C_6H_4$ | 2 | 2 |
| XXXV | Benzol-1,3,4,6-tetryl | $4\text{-}(CO_2C_2H_5)\text{-}C_6H_4$ | $4\text{-}CH_3\text{-}C_6H_4$ | 2 | 2 |
| Zusammenstellung ausgewählter Verbindungen der Formel (I) | | | | | |

Analytische Daten:
Analytische Daten:

I, $C_{19}H_{17}N_3O_5S_2$, M = 431.5, *N*-Phenyl-2-(3-(phenylsulfonyl)ureido)benzolsulfonamid

MS (ESI):m/z (%) = 430.0 (13) $[M\text{-}H]^-$, 273.0 (100) $[M\text{-}H\text{-}Ar^2SO_2NH_2]^-$, 247.0 (6) $[M\text{-}H\text{-}Ar^2SO_2NCO]^-$.
$^1H$ NMR (500 MHz, DMSO-$d_6$): $\delta$(ppm) = 11.71 (1H, s), 10.37 (1H, s), 8.72 (1H, s), 8.04 - 7.97 (2H, m,), 7.77 (1H, dd, *J* = 8.3, 1.2 Hz), 7.73 (1H, dd, J = 8.1, 1.7 Hz) 7.71 (1H, tt, J = 7.4, 1.5 Hz), 7.67-7.62 (2H, m), 7.49 (1H, ddd, J = 8.3, 7.4, 1.6 Hz), 7.21 - 7.13 (3H, m), 7.02 - 6.97 (3H, m).
$^{13}C$ NMR (126 MHz, DMSO-$d_6$): $\delta$(ppm) =149,06 (NH$\underline{C}$(O)NH), 139.71, 136.65, 135.21, 133.62, 133.40, 131.67, 129.09, 129.04, 128.96, 128.82, 127.34, 125.52, 124.59, 120.54.

**II,** $C_{20}H_{19}N_3O_5S_2$, M = 445.5, *N*-(2-Tolyl)-2-(3-(phenylsulfonyl)ureido)benzolsulfonamid

MS (ESI):m/z (%) = 444.0 (20) $[M\text{-}H]^-$, 287.0 (100) $[M\text{-}H\text{-}Ar^2SO_2NH_2]^-$, 261.1(10) $[M\text{-}H\text{-}Ar^2SO_2NCO]^-$.
$^1H$ NMR (500 MHz, DMSO-$d_6$): $\delta$(ppm) = 11.73 (1H, s), 9.73 (1H, s), 8.74 (1H, s), 8.00 - 7.98 (2H, m), 7.83 (1H, dd, *J* = 8.5, 1.1 Hz), 7.70 (1H, tt, J = 7.4, 1.6 Hz), 7.66 - 7.60 (3H, m), 7.51 (1H, ddd, *J* = 8.3, 7.6, 1.7 Hz), 7.13 (1H, ddd, *J* = 7.6, 1.1 Hz),7,05 (1H, ddd, J = 8.1, 6.5, 2.2 Hz) , 7.03 - 6.97 (3H, m), 1.95 (3H, s).
$^{13}C$ NMR (126 MHz, DMSO-$d_6$): $\delta$(ppm) = 148.96 (NH$\underline{C}$(O)NH), 139.78, 135.17, 134.24, 133.72, 133.57, 133.35, 130.68, 129.01, 128.90, 128.43, 127.31, 126.81, 126.56, 126.29, 123.41, 122.77, 17.24 ($\underline{C}H_3$).

**III,** $C_{20}H_{19}N_3O_5S_2$, M = 445.5, *N*-(4-Tolyl)-2-(3-(phenylsulfonyl)ureido) benzolsulfonamid

MS (ESI):m/z (%) = 287.0 (100) $[M\text{-}H\text{-}Ar^2SO_2NH_2]^-$, 261.1(5) $[M\text{-}H\text{-}Ar^2SO_2NCO]^-$.
$^1H$ NMR (500 MHz, DMSO-$d_6$): $\delta$(ppm) = 11.74 (1H, s), 10.23 (1H, s), 8.75 (1H, s), 8.04 - 7.99 (2H, m), 7.78 (1H, dd, *J* = 8.4, 1.1 Hz), 7.71 (1H, dd, 8.0,1.6 Hz),7.70 (1H, tt, J = 7.4, 1.5 Hz), 7.66 - 7.61 (2H, m), 7.46 (1H, ddd, *J* = 8.4, 7.4, 1.6 Hz), 7.14 (1H, ddd, *J* = 7.8, 7.5, 1.2 Hz), 7.00 - 6.94 (2H, m), 6.93 - 6.89 (2H, m), 2.14 (3H, s).

$^{13}C$ NMR (126 MHz, DMSO-$d_6$): δ(ppm) = 149.11 (NHC(O)NH), 139.81, 135.28, 134.11, 133.96, 133.54, 133.38, 129.58, 129.06, 129.01, 127.88, 127.35, 123.66, 123.32, 121.14, 20.25 ($CH_3$).

**IV,** $C_{20}H_{19}N_3O_6S_2$, M = 461.5, *N*-(4-Methoxyphenyl)-2-(3-(phenylsulfonyl)ureido) benzolsulfonamid

MS (ESI):m/z (%) = 460.0 (25) $[M-H]^-$, 303.0 (100) $[M-H-Ar^2SO_2NH_2]^-$, 278.0 (4) $[M-H-Ar^2SO_2NCO]^-$.
$^1H$ NMR (500 MHz, DMSO-$d_6$): δ(ppm) = 11.75 (1H, s), 10.06 (1H, s), 8.76 (1H, s), 8.02 - 7.99 (2H, m), 7.78 (1H, dd, *J* = 8.4, 1.2 Hz), 7.70 (1H, tt, J = 7.4, 1.6 Hz), 7.67-7.61 (3H,m), 7.47 (1H, ddd, *J* = 8.4, 7.4, 1.6 Hz), 7.14 (1H, ddd, *J* = 7.9, 7.4, 1.2 Hz), 6.94 - 6.90 (2H, m), 6.77 - 6.72 (2H, m), 3.65 (3H, s).
$^{13}C$ NMR (126 MHz, DMSO-$d_6$): δ(ppm) = 156.95 (C-$OCH_3$), 149.14 (NHC(O)NH), 139.82, 135.27, 133.50, 133.37, 129.06, 129.04, 128.93, 127.78, 127.31, 124.02, 123.60, 123.22, 114.32, 55.09 ($OCH_3$).

**V,** $C_{22}H_{21}N_3O_7S_2$, M = 503.5, Ethyl 2-(2-(3-(phenylsulfonyl)ureido)phenylsulfonamido) benzoat MS (ESI):m/z (%) = 501.9 (4) $[M-H]^-$, 345.1(100) $[M-H-Ar^2SO_2NH_2]^-$, 319.0 (15) $[M-H-Ar^2SO_2NCO]^-$.

$^1H$ NMR (500 MHz, DMSO-$d_6$): δ(ppm) = 11.93 (1H, s), 10.73 (1H, s), 8.66 (1H, s), 8.00 - 7.96 (2H, m), 7.81 (1H, dd, *J* = 8.0, 1.6 Hz), 7.77 (2H, 2xdd, *J* = 8.2, 1.4 Hz), 7.69 (1H, tt, J = 7.4, 1.6 Hz), 7.64 - 7.59 (2H, m), 7.58 - 7.46 (3H, m), 7.53 (1H, ddd, *J* = 8.3, 7.4, 1.6 Hz), 7.49 (1H, ddd, *J* = 8.3, 7.4, 1.6 Hz), 7.18 (1H, ddd, *J* = 7.7, 7.8, 1.2 Hz), 7.12 (1H, ddd, J = 7.9, 7.4, 1.2 Hz), 4.18 (2H, q, *J* = 7.1 Hz), 1.23 (3H, t,*J* = 7.1 Hz).
$^{13}C$ NMR (126 MHz, DMSO-$d_6$): δ(ppm) = 166.80 ($CO_2R$), 149.07 (NHC(O)NH), 139.74, 138.05, 135.04, 134.32, 134.25, 133.38, 130.87, 129.37, 129.02, 127.24, 127.10, 125.52, 124.24, 123.89, 120.15, 117.87, 61.47 ($OCH_2CH_3$), 13.71($OCH_2CH_3$).

**VI,** $C_{21}H_{19}N_3O_7S_2$, M = 489.5, Methyl 4-(2-(3-(phenylsulfonyl)ureido)phenylsulfonamido) benzoat MS (ESI):m/z (%) = 488.0 (26) $[M-H]^-$, 331.0 (100) $[M-H-Ar^2SO_2NH_2]^-$, 305.0 (50) $[M-H-Ar^2SO_2NCO]^-$.

$^1H$ NMR (500 MHz, DMSO-$d_6$): δ(ppm) = 11.68 (1H, s), 10.97 (1H, s), 8.71 (1H, s), 8.03 - 7.99 (2H, m), 7.80 (1H, dd, *J* = 8.1, 1.6 Hz), 7.79 - 7.74 (3H, m), 7.71 (1H,tt, J = 7.4, 1.3 Hz), 7.67 - 7.62 (2H, m), 7.51 (1H, ddd, *J* = 8.3, 7.4, 1.6 Hz), 7.20 (1H, ddd, *J* = 7.7, 1.2 Hz), 7.16 - 7.12 (2H, m), 3.78 (3H, s), 3.35 (1H, q, *J* = 7.0 Hz), 1.07 (2H, t, *J* = 7.0 Hz).
$^{13}C$ NMR (126 MHz, DMSO-$d_6$): δ(ppm) = 165.47 ($CO_2R$), 149.13 (NHC(O)NH), 141.49, 139.70, 135.17, 133.95, 133.40, 130.52, 129.05, 129.00, 127.99, 127.34, 125.54, 124.87, 124.05, 118.43, 51.84 ($OCH_3$).

**VII,** $C_{19}H_{16}ClN_3O_5S_2$, M = 465.9, *N*-(3-Chlorophenyl)-2-(3-(phenylsulfonyl)ureido) benzolsulfonamid

MS (ESI):m/z (%) = 307.0 (100) $[M-H-Ar^2SO_2NH_2]^-$, 281.0 (26) $[M-H-Ar^2SO_2NCO]^-$.
$^1H$ NMR (500 MHz, DMSO-$d_6$): δ(ppm) = 11.70 (1H, s), 10.67 (1H, s), 8.71 (1H, s), 8.02 - 7.99 (2H, m), 7.77 (1H, dd, *J* = 8.4, 1.2 Hz), 7.76 (2H, dd, *J* = 8.0, 1.6 Hz) 7.70 (1H,tt, J = 7.4, 1.3 Hz), 7.66 - 7.61 (2H, m), 7.52 (1H, ddd, *J* = 8.4 7.3, 1.6 Hz), 7.22 (1H, ddd, J = 9.1,7.9, 1.7 Hz), 7.18 (1H, dd, *J* = 8.1 Hz), 7.08 (1H, dd, *J* = 2.1 Hz), 7.03 (1H, ddd, J = 8.0, 2.1, 0.9 Hz)), 6.97 (1H, ddd, *J* = 8.2, 2.1, 0.9 Hz).
$^{13}C$ NMR (126 MHz, DMSO-$d_6$): δ(ppm) = 149.10 (NHC(O)NH), 139.71, 138.33, 135.26, 133.90, 133.43, 133.39, 130.76, 129.04, 128.88, 127.80, 127.31, 124.30, 123.97, 123.83, 119.76, 118.5.

**VIII,** $C_{26}H_{23}N_5O_6S_2$, M = 565.6, 2-(3-(Phenylsulfonyl)ureido)-*N*-(2-(3-phenylureido)phenyl)benzolsulfonamid

MS (ESI):m/z (%) = 564.1(100) $[M-H]^-$, 407.0 (80) $[M-H-Ar^2SO_2NH_2]^-$, 381.2 (12) $[M-H-Ar^2SO_2NCO]^-$.
$^1H$ NMR (500 MHz, DMSO-$d_6$): δ(ppm) = 11.74 (1H, s), 9.78 (1H, s), 9.47 (1H, s), 8.72 (1H, s), 8.00 (1H, dd, *J* = 8.3, 1.4 Hz), 8.00-7.97 (2H, m), 7.84 (1H, dd, J = 8.3, 1.2 Hz), 7.70 (1H, tt, J = 7.5, 1.6 Hz), 7.66 (1H, dd, J = 8.0, 1.6 Hz), 7.65-7.62 (2H, m), 7.58-7.54 (1H, m)7.54 - 7.51 (2H, m), 7.34 - 7.29 (2H, m), 7.17 (1H, ddd, *J* = 8.0, 7.2, 0.9 Hz), 7.05 (1H, ddd, *J* = 8.3, 7.4, 1.6 Hz), 7.00 (1H, tt, J = 7.4, 1.1Hz), 6.67 (1H, ddd, J = 7.8, 6.4, 1.5 Hz), 6.42 (1H, dd, *J* = 8.0, 1.5 Hz).
$^{13}C$ NMR (126 MHz, DMSO-$d_6$): δ(ppm) = 152.67(NHC(O)NH), 149.17(NHC(O)NH), 139.80, 139.67, 136.96, 135.56, 133.39, 131.70, 129.06, 128.85, 128.78, 128.74, 127.54, 127.33, 125.55, 124.14, 123.03, 122.18, 121.96, 121.15, 118.33, 118.31.

**IX,** $C_{20}H_{19}N_3O_5S_2$, M = 445.5, *N*-Phenyl-2-(3-tosylureido)benzolsulfonamid

MS (ESI):m/z (%) = 444.0 (22) $[M-H]^-$, 273.0 (100) $[M-H-Ar^2SO_2NH_2]^-$, 247.0 (9) $[M-H-Ar^2SO_2NCO]^-$.

$^{1}H$ NMR (500 MHz, DMSO-$d_6$): δ(ppm) = 11.65 (1H, s), 10.38 (1H, s), 8.72 (1H, s), 7.92 - 7.87 (2H, m), 7.79 (1H, dd, *J* = 8.4, 1.1 Hz), 7.74 (1H, dd, J = 8.0, 1.6 Hz), 7.48 (1H, ddd, *J* = 8.4, 7.3, 1.6 Hz), 7.44-7.40 (2H, m), 7.26 - 7.12 (3H, m), 7.05 - 7.01 (2H, m), 6.99 (1H, tt, J = 7.4, 1.4 Hz), 2.38 (3H, s).
$^{13}C$ NMR (126 MHz, DMSO-$d_6$): δ(ppm) = 149.11 (NHC(O)NH), 143.93, 136.91, 136.70, 135.31, 133.61, 129.46, 129.10, 128.97, 127.88, 127.45, 124.60, 123.69, 123.44, 120.54, 20.99 ($CH_3$).

**X,** $C_{21}H_{21}N_3O_5S_2$, M = 459.5, *N*-(2-Tolyl)-2-(3-tosylureido)benzolsulfonamid

MS (ESI):m/z (%) = 457.9 (1) $[M-H]^-$, 287.1 (100) $[M-H-Ar^2SO_2NH_2]^-$, 261.1(16) $[M-H-Ar^2SO_2NCO]^-$.
$^{1}H$ NMR (500 MHz, DMSO-$d_6$): δ(ppm) = 11.61 (1H, s), 9.69 (1H, s), 8.70 (1H, s), 7.87 - 7.80 (2H, m), 7.82 (1H, dd, *J* = 8.3, 1.1 Hz) 7.60 (1H, dd, *J* = 8.0, 1.6 Hz), 7.52 (1H, ddd, *J* = 8.4, 7.3, 1.6 Hz), 7.45 - 7.40 (2H, m), 7.25 - 7.19 (1H, m), 7.14 (1H, ddd, *J* = 8.0, 7.4, 1.1 Hz), 7.09 - 7.05 (1H, m), 7.03 - 7.00 (2H, m), 6.97 - 6.94 (1H, m), 2.40 (3H, s), 1.95 (3H, s)
$^{13}C$ NMR (126 MHz, DMSO-$d_6$): δ(ppm) = 149.00(NHC(O)NH), 143.85, 136.95, 135.23, 134.22, 133.74, 133.56, 130.69, 129.42, 128.90, 128.38, 127.36, 126.78, 126.48, 126.28, 123.35, 122.74, 20.98 ($CH_3$), 17.26 ($CH_3$).

**XI,** $C_{21}H_{21}N_3O_5S_2$, M = 459.5, *N*-(4-Tolyl)-2-(3-tosylureido)benzolsulfonamid

MS (ESI):m/z (%) = 287.1(100) $[M-H-Ar^2SO_2NH_2]^-$, 261.1(8) $[M-H-Ar^2SO_2NCO]^-$.
$^{1}H$ NMR (500 MHz, DMSO-$d_6$): δ(ppm) = 11.63 (1H, s), 10.20 (1H, s), 8.71 (1H, s), 7.90 - 7.86 (2H, m), 7.79 (1H, dd, *J* = 8.4, 1.2 Hz), 7.70 (1H, dd, *J* = 8.0, 1.6 Hz), 7.47 (1H, ddd, *J* = 8.4, 7.4, 1.6 Hz), 7.44 - 7.41 (2H, m), 7.14 (1H, ddd, *J* = 8.0, 7.3, 1.2 Hz), 6.97 - 6.94 (2H, m,), 6.91 - 6.88 (2H, m), 2.39 (3H, s), 2.15 (3H, s).
$^{13}C$ NMR (126 MHz, DMSO-$d_6$): δ(ppm) = 149.06 (NHC(O)NH), 143.84, 136.95, 135.33, 134.00, 133.95, 133.48, 129.52, 129.44, 128.96, 127.78, 127.41, 123.55, 123.23, 121.05, 20.95($CH_3$), 20.20($CH_3$).

**XII,** $C_{21}H_{21}N_3O_5S_2$, M = 459.5, *N*-(3-Tolyl)-2-(3-tosylureido)benzolsulfonamid

MS (ESI):m/z (%) = 58.0 (1) $[M-H]^-$, 287.1 (100) $[M-H-Ar^2SO_2NH_2]^-$, 261.0(10) $[M-H-Ar^2SO_2NCO]^-$.
$^{1}H$ NMR (500 MHz, DMSO-$d_6$): δ(ppm) = 11.64 (1H, s), 10.29 (1H, s), 8.72 (1H, s), 7.90 - 7.86 (2H, m), 7.79 (1H, dd, *J* = 8.4, 1.1 Hz), 7.74 (1H, dd, *J* = 8.0, 1.6 Hz), 7.48 (1H, ddd, *J* = 8.4, 7.4, 1.6 Hz), 7.42 (2H, d, *J* = 8.1 Hz), 7.17 (1H, ddd, *J* = 7.7, 1.2 Hz), 7.03 (1H, t, *J* = 7.8 Hz), 6.86 (1H, t, *J* = 1.9 Hz), 6.82 - 6.78 (2H, m), 2.38 (3H, s), 2.15 (3H, s).
$^{13}C$ NMR (126 MHz, DMSO-$d_6$): δ(ppm) = 149.05 (NHC(O)NH), 143.89, 138.55, 136.92, 136.60, 135.36, 133.56, 129.44, 128.89, 127.84, 127.39, 125.37, 123.63, 123.23, 121.12, 117.65, 20.97($CH_3$), 20.85($CH_3$).

**XIII,** $C_{21}H_{21}N_3O_6S_2$, M = 475.5, *N*-(4-Methoxyphenyl)-2-(3-tosylureido)benzolsulfonamid

MS (ESI):m/z (%) = 473.9 (2) $[M-H]^-$, 303.0 (100) $[M-H-Ar^2SO_2NH_2]^-$, 277.0 (5) $[M-H-Ar^2SO_2NCO]^-$.
$^{1}H$ NMR (500 MHz, DMSO-$d_6$): δ(ppm) = 11.64 (1H, s), 10.03 (1H, s), 8.72 (1H, s), 7.89 - 7.85 (2H, m), 7.79 (1H, dd, *J* = 8.4, 1.1 Hz), 7.64 (1H, dd, *J* = 8.0, 1.6 Hz), 7.48 (1H, ddd, *J* = 8.4, 7.3, 1.6 Hz), 7.44 - 7.40 (2H, m), 7.14 (1H, ddd, *J* = 7.9, 7.5, 1.2 Hz), 6.93 - 6.88 (2H, m), 6.75 - 6.71 (2H, m) 3.66 (3H, s), 2.39 (3H, s).
$^{13}C$ NMR (126 MHz, DMSO-$d_6$): δ(ppm) = 156.89 ($C-OCH_3$), 149.10 (NHC(O)NH), 143.84, 136.96, 135.32, 133.45, 129.45, 129.42, 128.92, 127.68, 127.36, 123.94, 123.50, 123.13, 114.26, 55.04 ($OCH_3$), 20.95($CH_3$).

XIV, $C_{23}H_{23}N_3O_7S_2$, M = 517.6, Ethyl 2-(2-(3-tosylureido)phenylsulfonamido)benzoat

MS (ESI):m/z (%) = 516.0 (5) $[M-H]^-$, 345.0 (100) ($[M-H-Ar^2SO_2NH_2]^-$, 319.1 (23) $[M-H-Ar^2SO_2NCO]^-$.
$^{1}H$ NMR (500 MHz, DMSO-$d_6$): δ(ppm) = 11.84 (1H, s), 10.73 (1H, s), 8.64 (1H, s), 7.87 - 7.84 (2H, m), 7.82 (1H, dd, *J* = 8.0, 1.7 Hz), 7.78 (1H, dd, *J* = 8.2, 1.1 Hz), 7.77 (1H, dd, *J* = 7,9, 1.6 Hz), 7.53 (1H, ddd, *J* = 8.3, 7.5, 1.6 Hz), 7.49 (1H, ddd, *J* = 8.4, 7.2, 1.7 Hz), 7.41 (1H, dd, *J* = 8.3, 1.1 Hz), 7.40 - 7.38 (2H, m), 7.18 (1H, ddd, *J* = 7.8, 7.6, 1.2 Hz), 7.13 (1H, ddd, *J* = 7.8, 7.5, 1.3 Hz) , 4.19 (2H, q, *J* = 7.1 Hz), 2.37 (3H, s), 1.24 (3H, t, *J* = 7.1 Hz).
$^{13}C$ NMR (126 MHz, DMSO-$d_6$): δ(ppm) = 166.78 ($CO_2R$), 149.07 (NHC(O)NH), 143.86, 138.08, 136.91, 135.09, 134.29, 134.23, 130.85, 129.39, 129.33, 127.31, 127.07, 124.17, 123.86, 123.82, 120.06, 117.81, 61.45 ($OCH_2CH_3$), 20.93($CH_3$), 13.70 ($OCH_2CH_3$).

**XV,** $C_{22}H_{21}N_3O_7S_2$, M = 503.5, Methyl 4-(2-(3-tosylureido)phenylsulfonamido)benzoat

MS (ESI):m/z (%) = 502.0 (1) $[M-H]^-$, 331.0 (100) $[M-H-Ar^2SO_2NH_2]^-$, 305.1(73) $[M-H-Ar^2SO_2NCO]^-$.
$^1H$ NMR (500 MHz, DMSO-$d_6$): δ(ppm) = 11.55 (1H, s), 10.93 (1H, s), 8.67 (1H, s), 7.90 - 7.86 (2H, m), 7.81 (1H, dd, *J* = 8.2, 1.6 Hz), 7.76 (1H, dd, *J* = 8.5, 1.2 Hz), 7.76 - 7.72 (2H, m), 7.51 (1H, ddd, *J* = 8.4, 7.3, 1.6 Hz), 7.43 - 7.41 (2H, m), 7.20(1H, ddd, *J* = 7.9, 7.4, 1.2 Hz), 7.09 (2H, m), 3.77 (3H, s), 2.39 (3H, s).
$^{13}C$ NMR (126 MHz, DMSO-$d_6$): δ(ppm) = 165.45 ($\underline{C}O_2R$), 149.13 (NH$\underline{C}$(O)NH), 143.97, 141.46, 136.80, 135.14, 133.96, 130.51, 129.47, 129.07, 127.99, 127.50, 124.81, 124.18, 124.05, 118.31, 51.84 (O$\underline{C}H_3$), 20.96($\underline{C}H_3$).

**XVI,** $C_{20}H_{18}ClN_3O_5S_2$, M = 479.9, N-(2-Chlorphenyl)-2-(3-tosylureido)benzolsulfonamid

MS (ESI):m/z (%) = 307.0 (100) $[M-H-Ar^2SO_2NH_2]^-$, 281.0 (90) $[M-H-Ar^2SO_2NCO]^-$.
$^1H$ NMR (500 MHz, DMSO-$d_6$): δ(ppm) = 11.64 (1H, s), 10.00 (1H, s), 8.65 (1H, s), 7.89 (1H, dd, *J* = 8.5, 1.2 Hz), 7.87 - 7.83 (2H, m), 7.64 (1H, dd, *J* = 8.0, 1.6 Hz), 7.52 (1H, ddd, *J* = 8.5, 7.2, 1.5 Hz), 7.41 (2H, d, *J* = 8.0 Hz), 7.32 (1H, dd, *J* = 8.0, 1.5 Hz), 7.27 (1H, dd, *J* = 8.0, 1.7 Hz), 7.22 (1H, ddd, *J* = 7.7, 1.5 Hz), 7.13 (1H, ddd, *J* = 8.1,7.2, 1.0 Hz), 7.12 (1H, ddd, *J* = 7.7, 1.2 Hz), 2.38 (3H, s)
$^{13}C$ NMR (126 MHz, DMSO-$d_6$): δ(ppm) = 148.94 (NH$\underline{C}$(O)NH), 143.89, 136.86, 135.37, 133.89, 132.51, 129.81, 129.43, 129.01, 128.96, 127.94, 127.90, 127.69, 127.38, 127.25, 123.34, 122.73, 20.99($\underline{C}H_3$).

**XVII,** $C_{20}H_{18}ClN_3O_5S_2$, M = 479.9, N-(3-Chlorphenyl)-2-(3-tosylureido)benzolsulfonamid

MS (ESI):m/z (%) = 477.9 (2) $[M-H]^-$, 307.0 (100) $[M-H-Ar^2SO_2NH_2]^-$, 281.0 (40) $[M-H-Ar^2SO_2NCO]^-$.
$^1H$ NMR (500 MHz, DMSO-$d_6$): δ(ppm) = 11.63 (1H, s), 10.68 (1H, s), 8.70 (1H, s), 7.90 - 7.87 (2H, m), 7.78 (1H, dd, *J* = 8.5, 1.2 Hz), 7.76 (1H, dd, *J* = 8.0, 1.6 Hz), 7.51 (1H, ddd, *J* = 8.4, 7.4, 1.6 Hz), 7.43 - 7.39 (2H, m), 7.19 (1H, ddd, *J* = 8.1, 7.4, 1.2 Hz), 7.18 (1H, dd, *J* = 8.2 Hz), 7.08 (1H, dd, *J* = 2.1 Hz), 7.04 (1H, ddd, *J* = 8.1, 2.1, 0.9 Hz), 6.99 (1H, ddd, *J* = 8.2, 2.2, 0.9 Hz), 2.37 (3H, s)
$^{13}C$ NMR (126 MHz, DMSO-$d_6$): δ(ppm) = 149.14 (NH$\underline{C}$(O)NH), 143.91, 138.37, 136.90, 135.35, 133.88, 133.46, 130.75, 129.45, 128.90, 127.77, 127.40, 124.29, 123.90, 123.81, 119.76, 118.55, 20.98($\underline{C}H_3$).

**XVIII,** $C_{20}H_{18}ClN_3O_5S_2$, M = 479.9, *N*-(4-Chlorphenyl)-2-(3-tosylureido)benzolsulfonamid

MS (ESI):m/z (%) = 307.0 (100) $[M-H-Ar^2SO_2NH_2]^-$, 281.0 (23) $[M-H-Ar^2SO_2NCO]^-$.
$^1H$ NMR (500 MHz, DMSO-$d_6$): δ(ppm) = 11.63 (1H, s), 10.56 (1H, s), 8.72 (1H, s), 7.91 - 7.86 (2H, m), 7.78 (1H, dd, *J* = 8.4, 1.1 Hz), 7.72 (1H, dd, *J* = 8.0, 1.5 Hz), 7.50 (1H, ddd, *J* = 8.4, 7.4, 1.6 Hz), 7.42 (2H, d, *J* = 8.0 Hz), 7.22 - 7.15 (3H, m), 7.02- 7.00 (2H, m), 2.38 (3H, s) $^{13}C$ NMR (126 MHz, DMSO-$d_6$): δ(ppm) = 149.15 (NH$\underline{C}$(O)NH), 143.91, 136.88, 135.76, 135.27, 133.78, 129.48, 129.46, 129.07, 128.94, 128.70, 127.74, 127.43, 123.75, 121.92, 20.98($CH_3$).

**XIX,** $C_{22}H_{23}N_3O_6S_2$, M = 489.5, *N*-(4-Ethoxyphenyl)-2-(3-tosylureido)benzolsulfonamid

MS (ESI):m/z (%) = 488.0 (3) $[M-H]^-$, 317.1 (100) $[M-H-Ar^2SO_2NH_2]^-$.
$^1H$ NMR (500 MHz, DMSO-$d_6$): δ(ppm) = 11.66 (1H, s), 10.03 (1H, s), 8.74 (1H, s), 7.90 - 7.86 (2H, m), 7.80 (1H, dd, *J* = 8.4, 1.2 Hz), 7.65 (1H, dd, *J* = 8.0, 1.6 Hz), 7.48 (1H, ddd, *J* = 8.4, 7.4, 1.6 Hz), 7.45 - 7.41 (2H, m), 7.14 (1H, ddd, *J* = 7.9, 7.5, 1.2 Hz), 6.93 - 6.88 (2H, m), 6.75 - 6.71 (2H, m), 3.91 (2H, q, *J* = 7.0 Hz), 2.39 (3H, s), 1.26 (3H, t, *J* = 7.0 Hz).
$^{13}C$ NMR (126 MHz, DMSO-$d_6$): δ(ppm) = 156.19 ($\underline{C}$-$OC_2H_5$), 149.11(NH$\underline{C}$(O)NH), 143.82, 136.97, 135.33, 133.43, 129.43, 128.98, 128.81, 127.67, 127.36, 123.92, 123.46, 123.08, 114.73, 63.05(O$\underline{C}H_2CH_3$), 20.94($\underline{C}H_3$), 14.48 ($OCH_2\underline{C}H_3$).

**XX,** $C_{21}H_{21}N_3O_6S_2$, M = 475.5, *N*-(2-Methoxyphenyl)-2-(3-tosylureido)benzolsulfonamid

MS (ESI):m/z (%) = 473.9 (3) $[M-H]^-$, 303.0 (100) $[M-H-Ar^2SO_2NH_2]^-$, 277.0 (10) $[M-H-Ar^2SO_2NCO]^-$.
$^1H$ NMR (500 MHz, DMSO-$d_6$): δ(ppm) = 11.68 (1H, s), 9.42 (1H, s), 8.61 (1H, s), 7.96 (1H, dd, *J* = 8.5, 1.2 Hz), 7.92 - 7.86 (2H, m), 7.58 (1H, dd, *J* = 7.9, 1.6 Hz), 7.47 (1H, ddd, *J* = 8.5, 7.4, 1.6 Hz), 7.43 (2H, d, *J* = 8.1 Hz), 7.24 (1H, dd, *J* = 7.8, 1.6 Hz), 7.07 (1H, dd, *J* = 5.1, 1.6 Hz), 7.06 (1H, ddd, *J* = 7.8, 5.9, 1.8 Hz), 6.86 - 6.78 (2H, m), 3.33 (3H, s), 2.38 (3H, s)
$^{13}C$ NMR (126 MHz, DMSO-$d_6$): δ(ppm) = 152.30 ($\underline{C}$-$OCH_3$), 148.97 (NH$\underline{C}$(O)NH), 143.97, 136.85, 135.40, 133.56, 129.45, 129.13, 127.60, 127.44, 127.17, 125.22, 124.05, 122.82, 121.92, 120.36, 111.56, 55.22 (O$\underline{C}H_3$), 20.97($\underline{C}H_3$).

**XXI,** $C_{27}H_{25}N_3O_8S_3$, M = 615.7, 3-(2-(3-Tosylureido)phenylsulfonamido)phenyl 4-methylbenzolsulfonat

MS (ESI):m/z (%) = 614.1(100) $[M\text{-}H]^-$, 443.0 (95) $[M\text{-}H\text{-}Ar^2SO_2NH_2]^-$, 417.0 (25) $[M\text{-}H\text{-}Ar^2SO_2NCO]^-$.
$^1H$ NMR (500 MHz, DMSO-$d_6$): δ(ppm) = 11.64 (1H, s), 10.70 (1H, s), 8.68 (1H, s), 7.91 - 7.85 (2H, m), 7.81 (1H, d, *J* = 8.3 Hz), 7.64 (3H,m), 7.52 (1H, ddd, *J* = 8.5, 7.2, 1.5 Hz), 7.40 (4H, t, *J* = 8.3 Hz,), 7.18 (1H, ddd, *J* = 7.7, 1.2 Hz), 7.13 (1H, dd, *J* = 8.2, Hz), 6.93 (1H, dd, *J* = 8.1, 2.0 Hz), 6.89 (1H, dd, *J* = 2.2 Hz), 6.61 (1H, dd, *J* = 8.1, 2.3 Hz), 2.37 (6H, s).
$^{13}C$ NMR (126 MHz, DMSO-$d_6$): δ(ppm) = 149.32, 149.19(NHC(O)NH), 145.71, 143.88, 138.30, 136.93, 135.33, 133.85, 131.19, 130.46, 130.09, 129.43128.77, 127.97, 127.50, 127.37, 125.58, 123.56, 118.57, 117.58, 113.36, 21.08($CH_3$), 20.96($CH_3$).

**XXII,** $C_{27}H_{25}N_5O_6S_2$, M = 579.6, 4-Methyl-*N*-((4-(*N*-(2-(3-phenylureido)phenyl) sulfamoyl)phenyl)carbamoyl)benzolsulfonamid

MS (ESI):m/z (%) = 578.1(100) $[M\text{-}H]^-$, 407.1 (55), $[M\text{-}H\text{-}Ar^2SO_2NH_2]^-$, 381.0 (8) $[M\text{-}H\text{-}Ar^2SO_2NCO]^-$.
$^1H$ NMR (500 MHz, DMSO-$d_6$):δ(ppm) = 11.63 (1H, s), 9.75 (1H, s), 9.45 (1H, s), 8.69 (1H, s), 8.30 (1H, s), 8.00 (1H, dd, *J* = 8.4, 1.3 Hz), 7.84 (1H, dd, *J* = 8.4, 1.2 Hz), 7.86 - 7.83 (2H, m), 7.65 (1H, dd, *J* = 8.0, 1.6 Hz), 7.55 (1H, ddd, *J* = 8.6, 7.2, 1.7 Hz), 7.52-7.49 (2H, m), 7.44 - 7.40 (2H, m), 7.33 - 7.29 (2H, m), 7.16 (1H, ddd, *J* = 8.1, 7.2, 1.2 Hz), 7.07 (1H, ddd, *J* = 8.3, 7.4, 1.6 Hz), 7.00 (1H, tt, J = 7.4, 1.1Hz), 6.68 (1H, ddd, J = 7.7, 1.5 Hz), 6.40 (1H, dd, *J* = 8.0, 1.6 Hz), 2.40 (3H, s).
$^{13}C$ NMR (126 MHz, DMSO-$d_6$): δ(ppm) = 152.62 (NHC(O)NH, 149.17(NHC(O)NH), 146.49, 143.86, 141.76, 141.40, 139.63, 136.95, 135.55, 129.43, 129.19, 128.74, 128.70, 127.53, 127.35, 125.56, 124.16, 121.92, 118.54, 118.30, 118.28, 116.84, 20.82($CH_3$).

**XXIII,** $C_{23}H_{23}N_3O_7S_2$, M = 517.6, Ethyl 4-(2-(3-tosylureido)phenylsulfonamido)benzoat

MS (ESI):m/z (%) = 516.9 (4) $[M\text{-}H]^-$, 345.0 (100) $[M\text{-}H\text{-}Ar^2SO_2NH_2]^-$, 319.1(70) $[M\text{-}H\text{-}Ar^2SO_2NCO]^-$.
$^1H$ NMR (500 MHz, DMSO-$d_6$): δ(ppm) = 11.53 (1H, s), 10.91 (1H, s), 8.66 (1H, s), 7.92 - 7.87 (2H, m), 7.82 (1H, dd, *J* = 8.0, 1.6 Hz), 7.76 (1H, dd, J = 8.4,1.1 Hz), 7.77 - 7.72 (2H, m), 7.51 (1H, ddd, *J* = 8.3, 7.4, 1.6 Hz), 7.43 (2H, d, *J* = 8.1 Hz), 7.22 - 7.18 (1H, m), 7.13 - 7.10 (2H, m), 4.24 (3H, q, *J* = 7.2 Hz), 2.39 (3H, s), 1.26 (3H, t, *J* = 7.1 Hz)
$^{13}C$ NMR (126 MHz, DMSO-$d_6$):δ(ppm) = 164.94 ($CO_2R$), 149.14 (NHC(O)NH), 143.93, 141.38, 136.82, 135.14, 133.94, 130.45, 129.44, 129.13, 127.98, 127.52, 125.12, 124.18, 124.02, 118.31, 60.45 ($OCH_2CH_3$), 20.96($CH_3$), 14.06 ($OCH_2CH_3$).

**XXIV,** $C_{20}H_{19}N_3O_5S_2$, M = 445.5, *N*-Phenyl-3-(3-tosylureido)benzolsulfonamid

MS (ESI):m/z (%) = 444.0 (100) $[M\text{-}H]^-$, 273.1 (20) $[M\text{-}H\text{-}Ar^2SO_2NH_2]^-$, 247.0 (8) $[M\text{-}H\text{-}Ar^2SO_2NCO]^-$.
$^1H$ NMR (500 MHz, DMSO-$d_6$): δ(ppm) = 10.82 (1H, s), 10.29 (1H, s), 9.17 (1H, s), 8.05 - 8.02 (1H, m), 7.92 - 7.88 (2H, m), 7.46 (1H, m), 7.44 - 7.40 (4H, m), 7.22 - 7.17 (2H, m), 7.12 - 7.08 (2H, m), 6.99 (1H, tt, 7.4, 1.2 Hz), 2.36 (3H, s).
$^{13}C$ NMR (126 MHz, DMSO-$d_6$): δ(ppm) = 149.42 (NHC(O)NH), 143.95, 140.29, 138.90, 137.58, 137.01, 129.63, 129.48, 129.06, 127.53, 124.05, 122.91, 121.22, 120.13, 116.67, 21.01($CH_3$).

**XXV,** $C_{27}H_{25}N_5O_6S_2$, M = 579.6, *N*-(2-(3-Phenylureido)phenyl)-3-(3-tosylureido) benzolsulfonamid

MS (ESI):m/z (%) = 578.1 (95) $[M\text{-}H]^-$, 407.1 (100) $[M\text{-}H\text{-}Ar^2SO_2NH_2]^-$.
$^1H$ NMR (500 MHz, DMSO-$d_6$): δ(ppm) = 9.56 (1H, s), 9.47 (1H, s), 9.16 (1H, s), 8.29 (1H, s), 8.05 (1H, dd, *J* = 8.3, 1.4 Hz), 7.93 (1H, t, *J* = 2.0 Hz, dd), 7.89 (2H, d, *J* = 8.2 Hz), 7.53 ( 1H, dd, J = 8.0, 2.1 Hz), 7.52 - 7.49 (2H, m), 7.44 (1H, t, *J* = 8.1 Hz),7.44 (2H, d, J = 8.1 Hz) 7.33 (1H, ddd, J = 7,8, 1,4 Hz), 7.31 - 7.27 (2H, m), 7.16 (1H, ddd, *J* = 8.0, 1.6 Hz), 6.98 (1H, t),
*J* = 7.3 Hz), 6.76 (1H, ddd, *J* = 7.6, 1.5 Hz), 6.49 (1H, dd, *J* = 8.0, 1.6 Hz), 2.39 (3H, s)
$^{13}C$ NMR (126 MHz, DMSO-$d_6$): δ(ppm) = 152.49(NHC(O)NH), 149.38 (NHC(O)NH), 143.91, 139.94, 139.76, 138.78, 136.98, 136.95, 129.47, 129.44, 128.73, 127.58, 127.49, 127.13, 124.88, 122.94, 121.88, 121.84, 121.70, 120.95, 118.25, 117.06, 21.00 ($CH_3$).

**XXVI,** $C_{20}H_{19}N_3O_5S_2$, M = 445.5, *N*-Phenyl-4-(3-tosylureido)benzolsulfonamid

MS (ESI):m/z (%) = 444.1(100) [M-H]$^-$, 273.0 (40) [M-H-Ar$^2$SO$_2$NH$_2$]$^-$, 247.1(68) [M-H-Ar$^2$SO$_2$NCO]$^-$.
$^1$H NMR (500 MHz, DMSO-*d*$_6$): δ(ppm) = 10.88 (1H, s), 10.11 (1H, s), 9.18 (1H, s), 7.88-7.84 (2H, m), 7.68 - 7.64 (2H, m), 7.50 - 7.47 (2H, m), 7.40 (2H, d, *J* = 8.1 Hz), 7.22 - 7.17 (2H, m), 7.10 - 7.06 (2H, m), 6.99 (1H, tt, J = 7.4, 1.3 Hz), 2.36 (3H, s)
$^{13}$C NMR (126 MHz, DMSO-*d*$_6$): δ(ppm) = 149.25 (NHC(O)NH), 143.93, 142.02, 137.71, 136.83, 133.34, 129.44, 129.00, 127.92, 127.47, 123.93, 120.09, 118.51, 20.97 ($CH_3$).

**XXVII,** $C_{21}H_{21}N_3O_5S_2$, M = 459.5, *N*-(3-Tolyl)-4-(3-tosylureido)benzolsulfonamid

MS (ESI):m/z (%) = 458.0(100) [M-H]$^-$, 287.0 (23) [M-H-Ar$^2$SO$_2$NH$_2$]$^-$, 261.1 (47) [M-H-Ar$^2$SO$_2$NCO]$^-$.
$^1$H NMR (500 MHz, DMSO-*d*$_6$): δ(ppm) = 10.84 (1H, s), 10.04 (1H, s), 9.17 (1H, s), 7.89 - 7.84 (2H, m), 7.69 - 7.64 (2H, m), 7.51 - 7.47 (2H, m), 7.42 - 7.38 (2H, m), 7.07 (1H, t, *J* = 7.7 Hz), 6.92 - 6.86 (2H, m), 6.80 (1H, ddt, *J* = 7.4, 1.7, 0.9 Hz), 2.37 (3H, s), 2.17 (3H, s)
$^{13}$C NMR (126 MHz, DMSO-*d*$_6$): δ(ppm) = 149.26 (NHC(O)NH), 143.93, 141.99, 138.33, 137.67, 136.84, 133.45, 129.44, 129.41, 128.80, 127.91, 127.47, 124.61, 120.48, 118.51, 116.98, 20.95($CH_3$).20.93 ($CH_3$).

**XXVIII,** $C_{22}H_{21}N_3O_7S_2$, M = 503.5, Methyl 4-(4-(3-tosylureido)phenylsulfonamido) benzoat

MS (ESI):m/z (%) = 502.0(100) [M-H]$^-$, 331.0 (12) [M-H-Ar$^2$SO$_2$NH$_2$]$^-$, 305.1(45) [M-H-Ar$^2$SO$_2$NCO]$^-$.
$^1$H NMR (500 MHz, DMSO-*d*$_6$): δ(ppm) = 10.88 (1H, s), 10.70 (1H, s), 9.20 (1H, s), 7.86 (2H, d, *J* = 8.0 Hz), 7.81 (2H, d, *J* = 8.6 Hz), 7.74 (2H, d, *J* = 8.6 Hz), 7.51 (2H, d, *J* = 8.7 Hz), 7.39 (2H, d, *J* = 8.0 Hz), 7.21 (2H, d, *J* = 8.6 Hz), 3.76 (3H, s), 2.35 (3H, s).
$^{13}$C NMR (126 MHz, DMSO-*d*$_6$): δ(ppm) = 165.58 ($CO_2R$), 149.26(NHC(O)NH), 143.93, 142.40, 142.37, 136.81, 132.93, 130.49, 129.41, 128.00, 127.46, 124.35, 118.64, 118.16, 51.76($OCH_3$), 20.95($CH_3$).

**XXIX,** $C_{20}H_{18}ClN_3O_5S_2$, M = 479.9, *N*-(4-Chlorphenyl)-4-(3-tosylureido)benzolsulfonamid

MS (ESI):m/z (%) = 478.0(100) [M-H]$^-$, 306.9(18) [M-H-Ar$^2$SO$_2$NH$_2$]$^-$, 281.1(52) [M-H-Ar$^2$SO$_2$NCO]$^-$.
$^1$H NMR (500 MHz, DMSO-*d*$_6$): δ(ppm) = 10.86 (1H, s), 10.27 (1H, s), 9.19 (1H, s), 7.90 - 7.85 (2H, m), 7.70 - 7.65 (2H, m), 7.53 - 7.49 (2H, m), 7.40 (2H, d, *J* = 8.1 Hz), 7.29 - 7.23 (2H, m), 7.11 - 7.07 (2H, m), 2.35 (3H, s).
$^{13}$C NMR (126 MHz, DMSO-*d*$_6$): δ(ppm) = 149.28 (NHC(O)NH), 143.95, 142.24, 136.84, 136.73, 132.98, 129.43, 129.01, 128.16, 127.96, 127.50, 121.67, 118.59, 20.98($CH_3$).

**XXX,** $C_{27}H_{15}N_2O_6S_2$, M = 579.6, 4-Methyl-*N*-((4-(*N*-(4-(3-phenylureido) phenyl)sulfamoyl) phenyl)carbamoyl)benzolsulfonamid

MS (ESI):m/z (%) = 578.1(100) [M-H]$^-$, 407.0 (18) [M-H-Ar$^2$SO$_2$NH$_2$]$^-$, 381.0(4) [M-H-Ar$^2$SO$_2$NCO]$^-$.
$^1$H NMR (500 MHz, DMSO-*d*$_6$): δ(ppm) = 10.91 (1H, s), 9.43 (2H, d, *J* = 30.4 Hz), 9.22 (1H, s), 8.28 (1H, s), 8.01 (1H, dd, *J* = 8.2, 1.3 Hz), 7.89 (2H, d, *J* = 8.1 Hz), 7.61 (2H, d, *J* = 8.9 Hz), 7.51 (4H, m), 7.43 (2H, d, *J* = 8.1 Hz), 7.29 (2H, t, *J* = 7.8 Hz), 7.16 (1H, ddd, *J* = 7.8, 1.5 Hz), 6.97 (1H, t, *J* = 7.4 Hz), 6.78 (1H, ddd, *J* = 7.9, 1.4 Hz), 6.50 (1H, dd, J = 7.9, 1.4 Hz), 2.39 (3H, s).
$^{13}$C NMR (126 MHz, DMSO-*d*$_6$): δ(ppm) = 152.53(NHC(O)NH), 149.26(NHC(O)NH), 143.96, 142.18, 139.72, 136.86, 136.83, 133.00, 129.47, 129.43, 128.71, 128.39, 127.52. 125.17, 121.96, 121.85, 120.99, 118.29, 118.28, 118.26, 20.99 ($CH_3$).

**XXXI,** $C_{23}H_{23}N_3O_7S_2$, M = 517.6, Ethyl 4-(4-(3-tosylureido)phenylsulfonamido)benzoat

MS (ESI):m/z (%) = 516.0(100) [M-H]$^-$, 345.0 (12) [M-H-Ar$^2$SO$_2$NH$_2$]$^-$, 319.0(43) [M-H-Ar$^2$SO$_2$NCO]$^-$
$^1$H NMR (500 MHz, DMSO-*d*$_6$): δ(ppm) = 10.87 (1H, s), 10.69 (1H, s), 9.20 (1H, s), 7.89 - 7.84 (2H, m), 7.83 - 7.79 (2H, m), 7.77 - 7.73 (2H, m), 7.55 - 7.50 (2H, m), 7.39 (2H, d, *J* = 8.1 Hz), 7.25 - 7.20 (2H, m), 4.22 (2H, q, *J* = 7.1 Hz), 2.34 (3H, s), 1.24 (3H, t, *J* = 7.1 Hz)
$^{13}$C NMR (126 MHz, DMSO-*d*$_6$): δ(ppm) = 165.10 ($CO_2R$), 149.27(NHC(O)NH), 143.93, 142.41, 142.33, 136.83, 132.95, 130.45, 129.41, 128.03, 127.49, 124.68, 118.64, 118.20, 60.38 ($OCH_2CH_3$), 20.95($CH_3$), 14.07 ($OCH_2CH_3$).

**XXXII,** $C_{28}H_{27}N_5O_8S_3$, M = 657.7, *N,N'*-(((4-(N-phenylsulfamoyl)-1,3-phenylen) bis(azanediyl))bis(carbonyl))bis(4-methylbenzenesulfonamid)

MS (ESI):m/z (%) = 656.1 (100) [M-H]$^-$, 485.1 (60) [M-H-$Ar^2SO_2NH_2$]$^-$, 459.0 (2) [M-H-$Ar^2SO_2NCO$]$^-$, 314.0 (13) [M-H-2x $Ar^2SO_2NH_2$]$^-$, 288 (4) [M-H-$Ar^2SO_2NCO$- $Ar^2SO_2NH_2$]$^-$.

$^1$H NMR (500 MHz, ACN-$d_3$): δ(ppm) = 8.92 (1H, s), 8.64 (1H, s), 8.06 (1H, s), 7.94 (1H, s), 7.88 - 7.82 (5H, m), 7.59 (1H, d, *J*=8.8 Hz), 7.38 - 7.31 (4H, m), 7.15 (1H, dd), 7.14 - 7.10 (2H, m), 7.02 - 6.98 (1H, m), 6.98 - 6.95 (2H, m), 2.39 (3H, s), 2.37 (3H, s)

$^{13}$C NMR (126 MHz, ACN-$d_3$): δ(ppm) = 149.76(NH$\underline{C}$(O)NH), 149.61(NH$\underline{C}$(O)NH), 146.07, 146.06, 143.80, 137.48, 137.34, 137.29, 137.04, 131.79, 130.66, 130.58, 130.21, 128.68, 128.60, 126.74, 123.08, 122.39, 114.66, 113.28, 21.68($\underline{C}H_3$), 21.65($\underline{C}H_3$).

**XXXIII,** $C_{32}H_{28}N_6O_{10}S_4$, M = 784.8 , *N*$^1$,*N*$^3$-Diphenyl-4,6-bis(3-(phenylsulfonyl)ureido) benzol-1,3-disulfonamid

MS (ESI):m/z (%) = 783.1 (100) [M-H]$^-$, 626.0 (75) [M-H-$Ar^2SO_2NH_2$]$^-$, 600.0 (5) [M-H-$Ar^2SO_2NCO$]$^-$, 469.0 (50) [M-H-2x $Ar^2SO_2NH_2$]$^-$, 442.9 (8) [M-H-$Ar^2SO_2NCO$- $Ar^2SO_2NH_2$]$^-$.

$^1$H NMR (500 MHz, DMSO-*$d_6$*): δ(ppm) = 11.93 (2H, s), 10.45 (2H, s), 8.71 (2H, s), 8.48 (1H, s), 8.15 (1H, s), 7.88 - 7.84 (4H, m), 7.77-7.71 (2H, m), 7.61 - 7.55 (4H, m), 7.13 - 7.07 (4H, m), 7.00 - 6.92 (6H, m).

$^{13}$C NMR (126 MHz, DMSO-*$d_6$*): δ(ppm) = 148.46(NH$\underline{C}$(O)NH), 139.76, 139.36, 135.87, 133.58, 129.17, 129.12, 128.84, 127.41, 125.54, 121.12, 120.98, 119.09.

**XXXIV,** $C_{34}H_{32}N_6O_{10}S_4$, M = 812.9, *N*$^1$,*N*$^3$-Diphenyl-4,6-bis(3-tosylureido)benzol-1,3-disulfonamid MS (ESI):m/z (%) = 811.0(100) [M-H]$^-$, 640.0 (70) [M-H-$Ar^2SO_2NH_2$]$^-$, 614.1(8) [M-H-$Ar^2SO_2NCO$]$^-$.

$^1$H NMR (500 MHz, DMSO-*$d_6$*): δ(ppm) = 11.85 (2H, s), 10.44 (2H, s), 8.70 (2H, s), 8.52 (1H, s), 8.15 (1H, s), 7.88 - 7.83 (4H, m), 7.48-7.42 (4H, m), 7.13 - 7.07 (4H, m), 6.99-6.93 (6H, m), 2.43 (6H, s),

$^{13}$C NMR (126 MHz, DMSO-*$d_6$*): δ(ppm) = 148.44(NH$\underline{C}$(O)NH), 144.14, 139.81, 136.51, 135.88, 129.54, 129.22, 129.17, 127.49, 125.58, 121.01, 120.92, 119.08, 21.04($\underline{C}H_3$).

**XXXV,** $C_{40}H_{40}N_6O_{14}S_4$, M = 957.03, Diethyl 4,4'-((4,6-bis(3-tosylureido)-1,3-phenylenedisulfonyl)bis(azanediyl))dibenzoat

MS (ESI):m/z (%) = 955.1(55) [M-H]$^-$, 784.1 (100) [M-H-$Ar^2SO_2NH_2$]$^-$, 758.1(7) [M-H-$Ar^2SO_2NCO$]$^-$, 613.0 (40) [M-H-2x $Ar^2SO_2NH_2$]$^-$, 587.0 (8) ([M-H-$Ar^2SO_2NCO$ -$Ar^2SO_2NH_2$]$^-$.

$^1$H NMR (500 MHz, DMSO-*$d_6$*): δ(ppm) = 11.80 (2H, s), 11.06 (2H, s), 8.77 (2H, s), 8.52 (1H, s), 8.28 (1H, s), 7.89-7.81 (4H, m), 7.71-7.65 (4H, m), 7.44-7.39 (4H, m), 7.04-6.98 (4H, m), 4.28 (4H, q, *J* = 7.0 Hz), 2.42 (6H, s), 1.30 (6H, t, *J* = 7.0 Hz).

$^{13}$C NMR (126 MHz, DMSO-*$d_6$*): δ(ppm) = 164.78($\underline{C}O_2R$), 148.51(NH$\underline{C}$(O)NH), 144.16, 140.61, 139.98, 136.43, 130.43, 129.55, 129.21, 127.50, 125.59, 125.53, 121.13, 118.44, 60.52 (O$\underline{C}H_2CH_3$), 21.01($\underline{C}H_3$), 14.04 ($OCH_2\underline{C}H_3$).

Der Auftrag einer wässrigen Auftragssuspension zur Ausbildung der wärmeempfindlichen farbbildenden Schicht eines wärmeempfindlichen Aufzeichnungspapiers erfolgte im Labormaßstabe mittels einer Stabrakel auf eine Seite eines synthetischen Basispapieres (Yupo® FP680) von 63 g/m$^2$. Nach Trocknung wurde ein thermisches Aufzeichnungsblatt erhalten. Die Auftragsmenge der wärmeempfindlichen farbbildenden Schicht lag zwischen 4,0 - 4,5 g/m$^2$.

Im Produktionsmaßstab erfolgte der Auftrag der wässrigen Auftragssuspension auf eine Papierbahn eines Flächengewichtes von 43 g/m$^2$ mittels des Curtain-Coating-Beschichtungsverfahrens. Die Viskosität der wässrigen Auftragssuspension betrug 450 mPas (nach Brookfield, 100 U/min, 20 °C) (im entlüfteten Zustand). Deren Oberflächenspannung betrug 46 mN/m (statistische Ringmethode). Das Streichwerk war in-line angeordnet. Das Curtain-Coating-Beschichtungsverfahren wurde bei einer Geschwindigkeit von 1550 m/min betrieben.

Nach dem Auftrag der wässrigen Auftragssuspension erfolgte in üblicher Weise der Trocknungsvorgang des beschichteten Papierträgers. Der Flächengewichtsauftrag der trockenen wärmeempfindlichen Schicht betrug 4,0 - 4,5 g/m$^2$.

Anhand der vorstehend gemachten Angaben wurde ein wärmeempfindliches Aufzeichnungsmaterial bzw. Thermopapier hergestellt, wobei die folgenden Rezepturen von wässrigen Auftragssuspensionen zur Ausbildung eines Verbundgebildes auf einem Trägersubstrat herangezogen und anschließend in üblicher Weise die weiteren Schichten, insbesondere eine Schutzschicht, ausgebildet wurden, worauf hier nicht gesondert eingegangen werden soll.

Herstellen der Dispersionen (jeweils für 1 Gew.-Teil) für die Auftragssuspensionen.

Die wässrige **Dispersion A** (Farbbildnerdispersion) wird hergestellt durch Mahlen von 20 Gew.-Teilen 3-N-n-Dibutylamin-6-methyl-7-anilinofluoran (ODB-2) mit 33 Gew.-Teilen einer 15 % wässrigen Lösung von Ghosenex™ L-3266 (sulfonierter Polyvinylalkohol, Nippon Ghosei) in einer Perlen-Mühle.

Die wässrige **Dispersion B** (Farbentwicklerdispersion) wird durch Mahlen von 40 Gew.-Teilen des Farbentwicklers zusammen mit 66 Gew.-Teilen einer 15 %igen wässrigen Lösung von Ghosenex™ L-3266 in der Perlen-Mühle hergestellt.

Die wässrige **Dispersion C** (Sensibilisierungsmittel-Dispersion) wurde hergestellt durch Mahlen von 40 Gew.-Teilen Sensibilisierungsmittel mit 33 Gew.-Teilen einer 15 %igen wässrigen Lösung von Ghosenex™ L-3266 in einer Perlen-Mühle.

Die wässrige **Dispersion D** (Alterungsschutzmittel- oder Stabilisatordispersion) wurde hergestellt durch Mahlen von 12,5 Gew.-Teilen UU (Urea-Urethane) mit 10 Gew.-Teilen einer 15 % wässrigen Lösung von Ghosenex™ L-3266 in einer Perlen-Mühle.

Alle durch Mahlen erzeugten Dispersionen haben eine mittlere Körngröße $D_{(4,3)}$ von 0,80 - 1,20 μm.

Die **Dispersion E** (Gleitmitteldispersion) ist eine 20 %ige Zinkstearat Dispersion, bestehend aus 9 Gew. Teilen Zn-Stearat, 1 Gew.Teil Ghosenex™ L-3266 und 40 Teilen Wasser.

Pigment **P** ist eine 72 %ige Streichkaolin-Suspension (Lustra® S, BASF).

Der **Binder** besteht aus einer 10%igen wässrigen Polyvinylalkohollösung (Mowiol 28-99, Kuraray Europe).

Die Auftragssuspension wird durch Mischen unter Rühren der Dispersionen entsprechend der Mengenangaben aus Tabelle 3 unter Berücksichtigung der Eintragsreihenfolge B, E, C, D, P, A, Binder hergestellt und mit Wasser auf einen Feststoffgehalt von ca. 25% gebracht.

Die Messung der Korngrößenverteilung der Auftragsdispersionen erfolgte durch Laserbeugung mit einem Coulter LS230-Gerät der Fa. Beckman Coulter.

| Rezeptur / Komponente | R1 | R2 |
|---|---|---|
| **A** | 1 | 1 |
| **B** | 1 | 1 |
| **C** | 1 | 1 |
| **D** | 0 | 1 |
| **E** | 56 | 56 |
| **P** | 146 | 126 |
| **Binder** | 138 | 138 |

Tabelle 3
Zusammenfassung der Rezepturen für die Auftragsdispersionen (Gew.-Teile)

Die so erhaltenen wärmeempfindlichen Beschichtungssuspensionen, wurden herangezogen, um Verbundgebilde aus Papierträger und Thermoreaktionsschicht herzustellen.

Die thermischen Aufzeichnungsmaterialien gemäß der Tabelle 3 wurden wie nachstehend beschrieben ausgewertet.

(1) Die Papierweisse strichseitig wurde nach DIN/ISO 2470 mit einem Elrepho 3000 Spektralphotometer bestimmt (Messunsicherheit der Weisse-Werte ≤0,5%).

(2) Dynamische Farbdichte:
Die Papiere (6 cm breite Streifen) wurden thermisch unter Verwendung des Atlantek 200 Testdruckers (Fa. Atlantek , USA) mit einer Kyocera Druckleiste von 200 dpi (dots per inch, 1 inch = 2,54 cm) und 560 Ohm bei einer angelegten Spannung von 20,6 V und einer maximalen Pulsbreite von 0,8 ms mit einem Schachbrett-Muster mit 10 Energieabstufungen bedruckt. Die Bilddichte (optische Dichte, opt. Dichte) wurde mit einem Macbeth-Densitometer RD-914, von Gretag gemessen, wobei der Wert "½" der o.D. bei der Energiestufe von 0,4 ms, der Wert "max."der Energiestufe von 0,8 ms entspricht. Die Messunsicherheit der o.D.-Werte wird mit ± 0,02 optische Dichteeinheiten veranschlagt.

(3) Statische Farbdichte (Starttemperatur):
Das Aufzeichnungsblatt wird gegen eine Reihe von auf unterschiedlichen Temperaturen erhitzen und thermostatierten metallischen Stempeln mit einem Anpressdruck von 0,2 kg/cm$^2$ und einer Kontaktzeit von 5 Sek gepresst (Thermoprüfer TP 3000QM, Maschinenfabrik Hans Rychiger AG, Steffisburg, Schweiz). Die Bilddichte (opt. Dichte) der so erzeugten Bilder wird mit einem Macbeth-Densitometer RD-914 von Gretag gemessen. Der statische Startpunkt ist definitionsmäßig die niedrigste Temperatur, bei welcher eine optische Dichte von 0,2 erreicht wird. Die Genauigkeit des Messverfahrens beträgt ≤ ±0,5 °C.

(4) Beständigkeitsprüfung des Druckbildes:

(4.1) Weichmacherbeständigkeit:
Auf die gemäß dem Verfahren von (2) dynamisch aufgezeichnete Probe des thermischen Aufzeichnungspapiers wurde eine weichmacherhaltigen Frischhaltefolie (PVC - Folie mit 20-25 % Dioctyladipat) unter Vermeiden von Falten und Lufteinschlüssen in Kontakt gebracht, zu einer Rolle gewickelt und 16 Std. gelagert. Eine Probe wird bei Raumtemperatur (20-22 °C), eine zweite bei 40°C gelagert. Nach Abziehen der Folie wurde die Bilddichte (opt. Dichte) gemessen und entsprechend der Formel (IV) in Bezug zu den entsprechenden Bilddichtewerten vor der WM-Einwirkung gesetzt.

(4.2) Beständigkeit gegenüber Haftkleber:
Auf die gemäß dem Verfahren von (2) dynamisch aufgezeichnete Probe des thermischen Aufzeichnungspapiers wurde je ein Streifen transparentes Selbstklebeband von Tesa (tesafilm® kristallklar, #57315) und getrennt davon ein Streifen Tesa Verpackungsklebeband (#04204) unter Vermeiden von Falten und Lufteinschlüssen geklebt. Nach Lagerung bei Raumtemperatur (20-22°C) wurde nach 24 Stunden die Bilddichte (opt. Dichte) - durch das jeweilige Klebeband- gemessen und entsprechend der Formel (IV) in Bezug zu den analog bestimmten Bilddichtewerten der frisch beklebten Musters gesetzt. Der so erhaltene Wert entspricht der % Abnahme der ursprünglichen Bildintensität.

$$\%\ Veränderung\ der\ o.D. = \left(\frac{Bilddichte\ \ nach\,Test}{Bilddichte\ vor\,Test} - 1\right) * 100 \qquad \text{(IV)}$$

Die Streuung der nach (IV) berechneten %-Werte beträgt ±2 Prozentpunkte.

LC-MS:Gerätetyp HPLC: Agilent 1200 Serie, Säule: Synergi 4µ Fusion-RP80A (250 x 4.6 mm, Phenomenex Inc.).
Eluent A : Acetonitril/Wasser+0,1% Ameisensäure (60/40, v/v).
Eluent B: Acetonitril+0,1% Ameisensäure.
Gradient: 0,0 min 100% A → 8,0 min 100% A (0.4 ml/min) → 10,0 min 100% A (1,0 ml/min) → 11 min 20% A (1.0 ml/min)→ 20,0 min 20% A (1.0 ml/min)→ 22,0 min 100% A (0.4 ml/min).

DAD Detektor G1315C (Agilent), gescannter Wellenlängenbereich 200-400 nm.

Single Quadrupole Mass Spectrometer,Typ 6120B (Agilent) mit Electrospray-Ionisierung (ESI), positiv- und negativ-Ionen-Modus, gescannter Massenbereich 100 - 1000 m/z.

Tabelle 4 fasst die Auswertung der gefertigten Aufzeichnungsmaterialien zusammen.

In Tabelle 5 sind für ausgewählte Entwicklerstoffe der erfindungsmäßigen Verbindungsklasse beispielhaft Möglichkeiten aufgezeigt, die Bildstabilität durch Zuhilfenahme von Alterungsschutzmitteln zu verbessern, ohne andere Leistungseigenschaften zu beeinträchtigen.

Tabelle 4 Auswertung der Papiermuster mit Rezeptur R1

| Nr. | Entwickler | Hintergrundweiße (%) | | opt. Dichte | | Statischer Startpunkt (°C) | Beständigkeit des Druckbildes | | | |
|---|---|---|---|---|---|---|---|---|---|---|
| | | | | | | | TESA, 24h, (bei max. o.D). | | WM-Folie, 16h, ( bei max. o.D.) | |
| | | 1 d R.T. | 7 d R.T. | ½ | max. | | #57315 | #04204 | RT | 40° |
| 1 | I | 87 | 87 | 1,36 | 1,35 | 80,5 | -43 | -70 | -7 | -40 |
| 2 | III | 89 | 89 | 1,26 | 1,32 | 85,5 | -38 | -67 | -9 | -76 |
| 3 | IV | 89 | 88 | 1,25 | 1,27 | 81,5 | -37 | -66 | -6 | -34 |
| 4 | VII | 86 | 86 | 1,28 | 1,31 | 76,5 | -36 | -60 | -5 | -27 |
| 5 | X | 89 | 90 | 1,25 | 1,27 | 85,0 | -45 | -70 | -9 | -63 |

(fortgesetzt)

| Nr. | Entwickler | Hintergrundweiße (%) | | opt. Dichte | | Statischer Startpunkt (°C) | Beständigkeit des Druckbildes | | | |
|---|---|---|---|---|---|---|---|---|---|---|
| | | | | | | | TESA, 24h, (bei max. o.D). | | WM-Folie, 16h, ( bei max. o.D.) | |
| | | 1 d R.T. | 7 d R.T. | ½ | max. | | #57315 | #04204 | RT | 40° |
| 6 | XII | 89 | 89 | 1,27 | 1,29 | 81,0 | -37 | -60 | -6 | -40 |
| 7 | XV | 89 | 89 | 1,30 | 1,30 | 81,5 | -25 | -48 | -5 | -20 |
| 8 | XVIII | 88 | 88 | 1,32 | 1,31 | 78,0 | -37 | -57 | -8 | -42 |
| 9 | XXIII | 89 | 89 | 1,25 | 1,29 | 81,5 | -26 | -50 | -5 | -26 |
| 10 | XXXII | 87 | 87 | 1,15 | 1,20 | 80,0 | -14 | -13 | -4 | -10 |
| 11 | XXXV | 87 | 87 | 1,23 | 1,26 | 82,5 | -6 | -5 | -2 | -9 |
| 12 | FE A* | 88 | 88 | 1,26 | 1,29 | 76,5 | -32 | -57 | -5 | -31 |
| 13 | FE B* | 89 | 89 | 1,25 | 1,28 | 85,5 | -50 | -70 | -15 | -91 |
| *nicht-phenolische Entwickler des Std. d. Technik | | | | | | | | | | |

Tabelle 5 Auswertung der Papiermuster ohne/mit Alterungsschutz (AS) mit Rezeptur R2

| Nr. | Entwickler | Hintergrundweiße (%) | | opt. Dichte | | Statischer Startpunkt (°C) | Beständigkeit des Druckbildes | | | |
|---|---|---|---|---|---|---|---|---|---|---|
| | | | | | | | TESA, 24h, (bei max. o.D). | | WM-Folie, 16h, ( bei max. o.D.) | |
| | | 1 Tag R.T. | 7 Tage R.T. | ½ | max. | | #57315 | #04204 | RT | 40° |
| 14 | II | 89 | 89 | 1,21 | 1,28 | 86 | -44 | -75 | -15 | -67 |
| 15 | II/AS | 90 | 88 | 1,21 | 1,21 | 83 | -32 | -59 | -4 | -18 |
| 16 | IX | 89 | 89 | 1,25 | 1,21 | 81 | -53 | -78 | -6 | -47 |
| 17 | IX/AS | 82 | 87 | 1,21 | 1,22 | 80 | -29 | -54 | -2 | -20 |
| 18 | XIV | 90 | 90 | 0,54 | 0,55 | 81 | -49 | -69 | -85 | -87 |
| 19 | XIV/AS | 88 | 90 | 0,84 | 0,95 | 78 | -43 | -45 | -13 | -40 |
| 20 | XXVI | 78 | 78 | 1,20 | 1,31 | 81 | -18 | -41 | -4 | -19 |
| 21 | XXVI/AS | 87 | 82 | 1,24 | 1,24 | 81 | -16 | -37 | -3 | -9 |

Das wärmeempfindliche Aufzeichnungsmaterial der vorliegenden Erfindung zeigt insbesondere die folgenden vorteilhaften Eigenschaften:

(1) Die Oberflächenweiße der erfindungsgemäßen wärmeempfindlichen Aufzeichnungspapiere ist sowohl im frischen Zustand, wie auch nach Lagerung, besser oder vergleichbar zu den Vergleichsmustern mit Farbentwicklern des Standes der Technik.

(2) Das aufgezeichnete Bild der erfindungsgemäßen wärmeempfindlichen Papiere mit dem erfindungsgemäßen Farbentwicklern weist eine Druckdichte ( optische Dichte-Werte " ½" und "max") auf, die in nichts den Farbentwicklern der Vergleichsmuster nachsteht.

(3) Die Temperatur ab welcher eine visuell merkliche Vergrauung der erfindungsgemäßen wärmeempfindlichen Aufzeichnungsmaterialien eintritt (statischer Startpunkt) ist vergleichbar oder höher als bei bekannten wärmeempfindlichen Aufzeichnungsmaterialien und erfüllt die Anforderungen an markttaugliche wärmeempfindliche Aufzeichnungsmaterialien. Ein hoher Startpunkt ist notwendig für Etikettierungsapplikationen im Lebensmittel-/Medizinbereich (thermische Laminierbarkeit, Mikrowellen- und Sterilisatortauglichkeit).

(4) Das Druckbild (max. opt. Dichte-Werte aus Tabelle 4), ist nach Einwirken von hydrophoben Agenzien (Klebstoffen, Weichmachern) kaum verblasst. Die Bildbeständigkeit ist besser oder vergleichbar als bei Einsatz von bekannten nichtphenolischen Farbentwicklern.

(5) Mit typischen Alterungsschutzmitteln kann im Bedarfsfalle die Bildbeständigkeit gegenüber hydrophoben Agenzien der erfindungsmäßigen Papiere gesteigert werden, ohne Nachteile hinsichtlich einer mangelhaften Oberflächenweiße oder niedriger Starttemperatur (Tab. 5).

(6) Mit dem erfindungsgemäßen Herstellungsverfahren lässt sich ein in allen wichtigen anwendungstechnischen Belangen hochwertiges wärmeempfindliches Aufzeichnungsmaterial unter wirtschaftlich vorteilhaften Bedingungen herstellen.

## Patentansprüche

1. Wärmeempfindliches Aufzeichnungsmaterial, umfassend ein Trägersubstrat sowie eine mindestens einen Farbbildner und mindestens einen phenolfreien Farbentwickler enthaltende wärmeempfindliche farbbildende Schicht, wobei der mindestens eine Farbentwickler eine Verbindung der Formel (I)

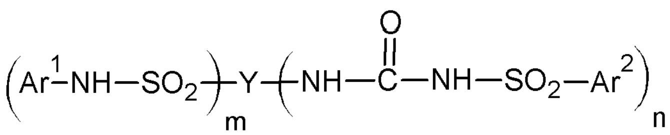

(I),

ist, wobei m und n unabhängig voneinander ≥ 1 sind, $Ar^1$ ein unsubstituierter oder substituierter (hetero) aromatischer Rest ist, $Ar^2$ ein unsubstituierter oder substituierter Phenyl-Rest ist und Y mindestens eine (m+n)-fach substituierte Benzol- oder Naphthalin-Gruppe ist.

2. Wärmeempfindliches Aufzeichnungsmaterial nach Anspruch 1, wobei m 1 oder 2 ist.

3. Wärmeempfindliches Aufzeichnungsmaterial nach mindestens einem der vorangegangenen Ansprüche, wobei n 1 oder 2 ist.

4. Wärmeempfindliches Aufzeichnungsmaterial nach mindestens einem der vorangegangenen Ansprüche, wobei $Ar^1$ ein unsubstituierter Phenyl-, ein unsubstituierter 1-Naphtyl-, ein unsubstituierter 2-Naphtyl-, ein einfach substituierter Phenyl-Rest, insbesondere ein 4-Alkoxycarbonylphenyl-Rest, ein einfach substituierter 1-Naphtyl- oder ein einfach substituierter 2-Naphtyl-Rest ist.

5. Wärmeempfindliches Aufzeichnungsmaterial nach Anspruch 4, wobei $Ar^1$ ein unsubstituierter Phenyl- oder ein einfach substituierter Phenyl-Rest ist.

6. Wärmeempfindliches Aufzeichnungsmaterial nach Anspruch 5, wobei der einfach substituierte Phenyl-Rest mit einem $C_1$-$C_5$-Alkyl-, einem Alkenyl-, einem Alkinyl-, einem Benzyl-, eine RO-, einem Halogen-, einem ROC-, einem $RO_2C$-, einem CN-, einem $NO_2$-, -R-$SO_2O$-, einem RO-$SO_2$-, einem R-NH-$SO_2$-, einem R-$SO_2$NH-, einem R-NH-CO-NH-, einem R-$SO_2$-NH-CO-NH-, einem R-NH-CO-NH-$SO_2$- oder einem R-CO-NH-Rest, wobei R ein $C_1$-$C_5$-Alkyl-, ein Alkenyl-, ein Alkinyl-, ein Phenyl-, ein Tolyl- oder ein BenzylRest, bevorzugt ein Phenyl- oder ein p-Tolyl-Rest, substituiert ist.

7. Wärmeempfindliches Aufzeichnungsmaterial nach mindestens einem der vorangegangenen Ansprüche, wobei $Ar^2$ ein unsubstituierter Phenyl- oder ein einfach substituierter Phenyl-Rest, insbesondere ein mit einem $C_1$-$C_4$-Alkyl-Rest, besonders bevorzugt mit einem Methyl-Rest, oder mit einem Halogen-Rest substituierter Phenyl-Rest ist.

8. Wärmeempfindliches Aufzeichnungsmaterial nach mindestens einem der vorangegangenen Ansprüche, wobei Y

eine (m+n)-fach substituierte Benzol- oder Naphthalin-Gruppe, vorzugsweise eine Benzol-Gruppe, ist.

**9.** Wärmeempfindliches Aufzeichnungsmaterial nach mindestens einem der vorangegangenen Ansprüche, wobei $Ar^1$ ein unsubstituierter Phenyl-Rest oder ein einfach substituierter Phenyl-Rest, $Ar_2$ ein unsubstituierter Phenyl-Rest oder ein einfach substituierter Phenyl-Rest und Y eine (m+n)-fach substituierte Benzol-Gruppe ist.

**10.** Wärmeempfindliches Aufzeichnungsmaterial nach mindestens einem der vorangegangenen Ansprüche, wobei der mindestens eine Farbbildner ein Farbstoff vom Triphenylmethan-Typ, vom Fluoran-Typ, vom Azaphthalid-Typ und/oder vom Fluoren-Typ, bevorzugt vom Fluoran-Typ, ist.

**11.** Wärmeempfindliches Aufzeichnungsmaterial nach mindestens einem der vorangegangenen Ansprüche, wobei neben der Verbindung der Formel (I) ein oder mehrere weitere nicht-phenolische Farbentwickler vorliegen.

**12.** Wärmeempfindliches Aufzeichnungsmaterial nach mindestens einem der vorangegangenen Ansprüche, wobei die Verbindung der Formel (I) nach mindestens einem der Ansprüche 1 bis 9 in einer Menge von etwa 3 bis etwa 35 Gew.-%, bevorzugt von etwa 10 bis etwa 25 Gew.-%, bezogen auf den gesamten Feststoffgehalt der wärmeempfindlichen Schicht, vorliegt.

**13.** Wärmeempfindliches Aufzeichnungsmaterial nach mindestens einem der vorangegangenen Ansprüche, wobei die wärmeempfindliche farbbildende Schicht eine Harnstoff-Urethan-Verbindung der allgemeinen Formel (II)

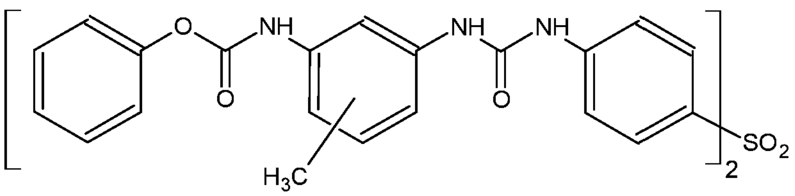

(II)

enthält.

**14.** Verfahren zur Herstellung eines wärmeempfindlichen Aufzeichnungsmaterials nach mindestens einem der vorangegangenen Ansprüche, wobei auf ein Trägersubstrat eine die Ausgangsmaterialien der wärmeempfindlichen farbbildenden Schicht enthaltende wässrige Suspension aufgetragen und getrocknet wird, wobei die wässrige Auftragssuspension einen Feststoffgehalt von etwa 20 bis etwa 75 Gew.-% , bevorzugt von etwa 30 bis etwa 50 Gew.%, aufweist, und mit dem Curtain-Coating-Beschichtungsverfahren bei einer Betriebsgeschwindigkeit der Streichanlage von mindestens etwa 400 m/min, bevorzugt von mindestens etwa 1000 m/min, ganz besonders bevorzugt von mindestens etwa 1500 m/min, aufgetragen und getrocknet wird.

**15.** Wärmeempfindliches Aufzeichnungsmaterial nach mindestens einem der Ansprüche 1 bis 13, erhältlich gemäß dem Verfahren nach Anspruch 14.

## Claims

**1.** A heat-sensitive recording material comprising a carrier substrate and a heat-sensitive, colour-forming layer containing at least one colour former and at least one phenol-free colour developer, wherein the at least one colour developer is a compound of the formula (I)

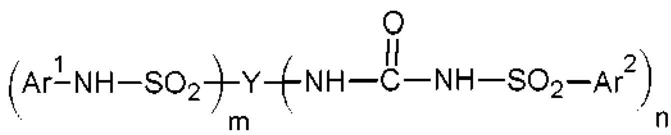

(I),

wherein m and n are mutually independently $\geq 1$, $Ar^1$ is an unsubstituted or substituted (hetero)aromatic residue, $Ar^2$ is an unsubstituted or substituted phenyl residue and Y is at least one (m+n)-fold substituted benzene or naphthalene group.

**2.** A heat-sensitive recording material according to Claim 1, wherein m is 1 or 2.

**3.** A heat-sensitive recording material according to at least one of the preceding claims, wherein n is 1 or 2.

**4.** A heat-sensitive recording material according to at least one of the preceding claims, wherein $Ar^1$ is an unsubstituted phenyl, an unsubstituted 1-naphthyl, an unsubstituted 2-naphthyl, a monosubstituted phenyl residue, in particular a 4-alkoxycarbonylphenyl residue, a monosubstituted 1-naphthyl or a monosubstituted 2-naphthyl residue.

**5.** A heat-sensitive recording material according to Claim 4, wherein $Ar^1$ is an unsubstituted phenyl or a monosubstituted phenyl residue.

**6.** A heat-sensitive recording material according to Claim 5, wherein the monosubstituted phenyl residue is substituted with a $C_1$-$C_5$ alkyl, an alkenyl, an alkynyl, a benzyl, an RO, a halogen, an ROC, an $RO_2C$, a CN, an $NO_2$, -R-$SO_2$O-, an RO-$SO_2$, an R-NH-$SO_2$, an R-$SO_2$NH, an R-NH-CO-NH, an R-$SO_2$-NH-CO-NH, an R-NH-CO-NH-$SO_2$ or an R-CO-NH residue, wherein R is a $C_1$-$C_5$ alkyl, an alkenyl, an alkynyl, a phenyl, a tolyl or a benzyl residue, preferably a phenyl or a p-tolyl residue.

**7.** A heat-sensitive recording material according to at least one of the preceding claims, wherein $Ar^2$ is an unsubstituted phenyl residue or a monosubstituted phenyl residue, in particular a phenyl residue substituted with a $C_1$-$C_4$ alkyl residue, particularly preferably with a methyl residue, or with a halogen residue.

**8.** A heat-sensitive recording material according to at least one of the preceding claims, wherein Y is an (m+n)-fold substituted benzene or naphthalene group, preferably a benzene group.

**9.** A heat-sensitive recording material according to at least one of the preceding claims, wherein $Ar^1$ is an unsubstituted phenyl residue or a monosubstituted phenyl residue, $Ar^2$ an unsubstituted phenyl residue or a monosubstituted phenyl residue and Y is an (m+n)-fold substituted benzene group.

**10.** A heat-sensitive recording material according to at least one of the preceding claims, wherein the at least one colour former is a dye of the triphenylmethane type, of the fluoran type, of the azaphthalide type and/or of the fluorene type, preferably of the fluoran type.

**11.** A heat-sensitive recording material according to at least one of the preceding claims, wherein one or more further non-phenolic colour developers are present in addition to the compound of formula (I).

**12.** A heat-sensitive recording material according to at least one of the preceding claims, wherein the compound of formula (I) according to at least one of Claims 1 to 9 is present in a quantity of approx. 3 to approx. 35 wt.%, preferably of approx. 10 to approx. 25 wt.%, based on the entire solids content of the heat-sensitive layer.

**13.** A heat-sensitive recording material according to at least one of the preceding claims, wherein the heat-sensitive colour-forming layer contains a urea-urethane compound of the general formula (II)

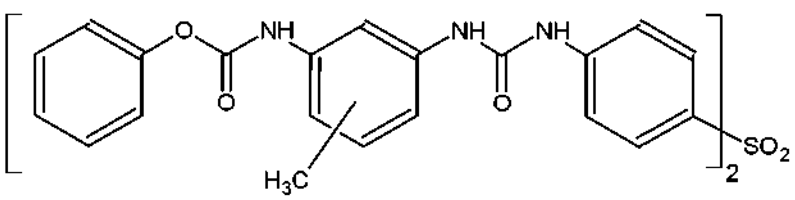

(II).

**14.** A method for producing a heat-sensitive recording material according to at least one of the preceding claims, wherein an aqueous suspension containing the starting materials of the heat-sensitive colour-forming layer is applied to a carrier substrate and dried, wherein the aqueous application suspension has a solids content of approx. 20 to approx. 75 wt.%, preferably of approx. 30 to approx. 50 wt.%, and is applied and dried using the curtain coating method at a coating line operating speed of at least approx. 400 m/min, preferably of at least approx. 1000 m/min, particularly preferably of at least approx. 1500 m/min.

**15.** A heat-sensitive recording material according to at least one of Claims 1 to 13 obtainable by the method according

to Claim 14.

## Revendications

**1.** Matériau d'enregistrement thermosensible, comprenant un substrat support ainsi qu'une couche thermosensible de formation de couleur contenant au moins un agent de formation de couleur et au moins un développeur de couleur, exempt de phénol, ledit au moins un développeur de couleur étant un composé de formule (I)

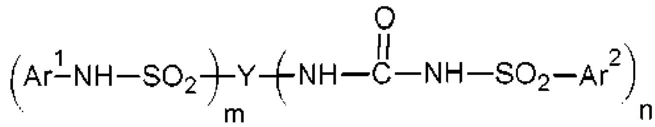

(I),

m et n indépendamment l'un de l'autre, étant $\geq 1$, $Ar^1$ représentant un radical (hétéro)aromatique non substitué ou substitué, $Ar^2$ représentant un radical phényle non substitué ou substitué et Y représentant au moins un groupe benzène ou naphtalène (m+n)-substitué.

**2.** Matériau d'enregistrement thermosensible selon la revendication 1, m valant 1 ou 2.

**3.** Matériau d'enregistrement thermosensible selon au moins l'une quelconque des revendications précédentes, n valant 1 ou 2.

**4.** Matériau d'enregistrement thermosensible selon au moins l'une quelconque des revendications précédentes, $Ar^1$ représentant un radical phényle non substitué, un radical 1-naphtyle non substitué, un radical 2-naphtyle non substitué, un radical phényle monosubstitué, en particulier un radical 4-alcoxycarbonylphényle, un radical 1-naphtyle monosubstitué ou un radical 2-naphtyle monosubstitué.

**5.** Matériau d'enregistrement thermosensible selon la revendication 4, $Ar^1$ représentant un radical phényle non substitué ou un radical phényle monosubstitué.

**6.** Matériau d'enregistrement thermosensible selon la revendication 5, le radical phényle monosubstitué étant substitué par un radical $C_1$-$C_5$-alkyle, un radical alcényle, un radical alcynyle, un radical benzyle, un radical RO, un radical halogène, un radical ROC, un radical $RO_2C$, un radical CN, un radical $NO_2$, un radical -R-$SO_2O$, un radical RO-$SO_2$, un radical R-NH-$SO_2$, un radical R-$SO_2NH$, un radical R-NH-CO-NH, un radical R-$SO_2$-NH-CO-NH, un radical R-NH-CO-NH- $SO_2$ ou un radical R-CO-NH, R représentant un radical $C_1$-$C_5$-alkyle, un radical alcényle, un radical alcynyle, un radical phényle, un radical toluyle ou un radical benzyle, de préférence un radical phényle ou un radical p-toluyle.

**7.** Matériau d'enregistrement thermosensible selon au moins l'une quelconque des revendications précédentes, $Ar^2$ représentant un radical phényle non substitué ou un radical phényle monosubstitué, en particulier un radical phényle substitué par un radical $C_1$-$C_4$-alkyle, de manière particulièrement préférée par un radical méthyle, ou par un radical halogène.

**8.** Matériau d'enregistrement thermosensible selon au moins l'une quelconque des revendications précédentes, Y représentant un groupe benzène ou naphtalène (m+n)-substitué, de préférence un groupe benzène.

**9.** Matériau d'enregistrement thermosensible selon au moins l'une quelconque des revendications précédentes, $Ar^1$ représentant un radical phényle non substitué ou un radical phényle monosubstitué, $Ar^2$ représentant un radical phényle non substitué ou un radical phényle monosubstitué et Y représentant un groupe benzène (m+n)-substitué.

**10.** Matériau d'enregistrement thermosensible selon au moins l'une quelconque des revendications précédentes, ledit au moins un agent de formation de couleur étant un colorant du type triphénylméthane, du type fluorane, du type azaphtalide et/ou du type fluorène, de préférence du type fluorane.

**11.** Matériau d'enregistrement thermosensible selon au moins l'une quelconque des revendications précédentes, un ou plusieurs autres développeurs de couleur non phénoliques étant présents à côté du composé de formule (I).

**12.** Matériau d'enregistrement thermosensible selon au moins l'une quelconque des revendications précédentes, le composé de formule (I) selon au moins l'une quelconque des revendications 1 à 9 étant présent en une quantité d'environ 3 à environ 35 % en poids, de préférence d'environ 10 à environ 25 % en poids, par rapport à la teneur totale en solides de la couche thermosensible.

**13.** Matériau d'enregistrement thermosensible selon au moins l'une quelconque des revendications précédentes, la couche thermosensible de formation de couleur contenant un composé d'urée-uréthane de formule générale (II)

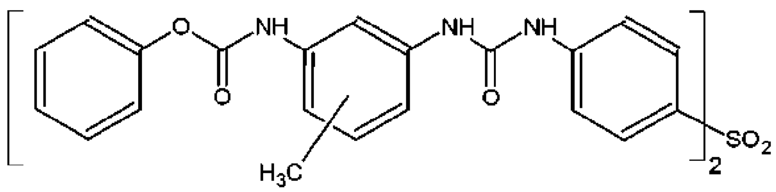

(II).

**14.** Procédé pour la préparation d'un matériau d'enregistrement thermosensible selon au moins l'une quelconque des revendications précédentes, une suspension aqueuse contenant les matériaux de départ de la couche thermosensible de formation de couleur étant appliquée sur un substrat support et séchée, la suspension aqueuse d'application présentant une teneur en solides d'environ 20 à environ 75 % en poids, de préférence d'environ 30 à environ 50 % en poids et étant appliquée par un procédé de revêtement en rideau à une vitesse de fonctionnement de l'installation d'enduction d'au moins environ 400 m/min, de préférence d'au moins environ 1000 m/min, de manière tout particulièrement préférée d'au moins environ 1500 m/min et séchée.

**15.** Matériau d'enregistrement thermosensible selon au moins l'une quelconque des revendications 1 à 13, pouvant être obtenu selon le procédé selon la revendication 14.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- EP 0526072 A1
- WO 0035679 A1
- EP 0535887 A1
- EP 0542556 A1
- EP 0604832 B1
- EP 0620122 A1
- EP 1044824 A2
- JP H0664335 B
- JP H0958242 B
- JP H11263067 B
- EP 0701905 A1
- DE 10196052 T1
- DE 102004029261 B4